# EUROPEAN PATENT SPECIFICATION

(11) **EP 4 041 164 B1**
(45) Date of publication and mention of the grant of the patent: **30.07.2025**
(21) Application number: 20792327.7
(22) Date of filing: 09.10.2020
(51) Int. Cl.: A61F 13/02

(54) **APPARATUSES AND METHODS FOR NEGATIVE PRESSURE WOUND THERAPY**
VORRICHTUNGEN UND VERFAHREN FÜR UNTERDRUCKWUNDTHERAPIE
APPAREILS ET PROCÉDÉS DE TRAITEMENT DE PLAIES PAR PRESSION NÉGATIVE

(30) Priority: 11.10.2019 GB 201914710
(43) Date of publication of application: 17.08.2022
(73) Proprietor: T.J. Smith and Nephew, Limited, Hull HU3 2BN (GB)
(72) Inventor: ASKEM, Ben, Alan, Hull HU3 2BN (GB); BEADLE, Victoria, Hull HU3 2BN (GB); CZECH, Maximilien, Henri, Hull HU3 2BN (GB); ELDER, David, Michael, Hull HU3 2BN (GB); FISHER, Adam, John, Hull HU3 2BN (GB); MADRIZ, Camilo, Patrick, Hull HU3 2BN (GB); PARRY, Luke, Michael, Hull HU3 2BN (GB); PHILLIPS, Marcus, Damian, Hull HU3 2BN (GB); SIDEBOTTOM, Hannah, Bailey, Hull HU3 2BN (GB); WEEDON, Fraser, George, Hull HU3 2BN (GB)
(74) Representative: Guy, Mark Robert
(86) International application number: PCT/EP2020/078376
(87) International publication number: WO 2021/069642

(56) References cited:
- WO-A1-2014/020440
- WO-A2-2013/175306

## Description

### BACKGROUND OF THE INVENTION

### Field of the Invention

Embodiments of the present disclosure relate to methods and apparatuses for dressing and treating a wound with reduced pressure therapy or topical negative pressure (TNP) therapy. In particular, but without limitation, embodiments disclosed herein relate to negative pressure therapy devices, methods for controlling the operation of TNP systems, and methods of using TNP systems.

### Description of the Related Art

Many different types of wound dressings are known for aiding in the healing process of a human or animal. These different types of wound dressings include many different types of materials and layers, for example, gauze, pads, foam pads or multi-layer wound dressings. Topical negative pressure (TNP) therapy, sometimes referred to as vacuum assisted closure, negative pressure wound therapy (NPWT), or reduced pressure wound therapy, is widely recognized as a beneficial mechanism for improving the healing rate of a wound. Such therapy is applicable to a broad range of wounds such as incisional wounds, open wounds and abdominal wounds or the like.

TNP therapy assists in the closure and healing of wounds by reducing tissue oedema, encouraging blood flow, stimulating the formation of granulation tissue, removing excess exudates and may reduce bacterial load and, thus, infection to the wound. Furthermore, TNP therapy permits less outside disturbance of the wound and promotes more rapid healing.

WO2013175306 discloses embodiments are directed towards wound dressings comprising a liquid and gas permeable transmission layer, an absorbent layer for absorbing wound exudate, the absorbent layer overlying the transmission layer, a gas impermeable cover layer overlying the absorbent layer and comprising a first orifice, wherein the cover layer is moisture vapor permeable. Some embodiments are directed to improved fluidic connectors or suction adapters for connecting to a wound site, for example using softer, kink-free conformable suction adapters.

WO2014020440 discloses embodiments directed to negative pressure treatment systems and wound dressing systems with an acquisition distribution layer that may be used for the treatment of wounds. In particular, some embodiments are directed to improved wound dressings comprising an obscuring layer that may hide fluid contained therein.

### SUMMARY OF THE INVENTION

Embodiments of the invention disclosed herein are directed to apparatuses, systems, devices and methods for use in negative pressure wound therapy. It will be understood by one of skill in the art that application of the materials, devices, methods, and systems described herein are not limited to a particular tissue or a particular injury. The invention is defined by appended claims. Embodiments or examples that are not covered by the scope of the appended claims, for examples methods for treating a wound site, are provided for understanding of the invention only.

According to the invention, a system to provide negative pressure to a wound site and a periwound area surrounding the wound site is provided according to claim 1.

In some embodiments, a system to provide negative pressure to a wound site is disclosed that may comprise an opening in the top layer that may be configured to transmit negative pressure to the wound area and the periwound site through the wound dressing and remove bodily fluids from the wound site and the periwound area. The one or more placement guides may be printed directly on the upper surface of the top layer or the one or more placement guides may be provided on a sticker that may be configured to be attached to the upper surface of the top layer. Some of the one or more placement guides may be directly printed on the upper surface of the top layer while the other one or more placement guides may be provided on a sticker that may be attached to the upper surface of the top layer. The one or more placement guides may comprise two substantially perpendicular lines that intersect at the opening and a circle with the opening at a center of the circle. The port may comprise one or more alignment features that may be configured to interact with the one or more placement guides of the top layer. In some embodiments, the intermediate layer comprises an acquisition distribution layer configured to communicate fluid through the wound dressing.

Some of the embodiments described herein provide a method for treating a wound site is disclosed. The method may comprise positioning a wound dressing over the wound site and a periwound area surrounding the wound site, positioning a port on the upper surface of the top layer, and applying negative pressure to the wound site and the periwound area through the wound dressing for a period of time. The wound dressing may comprise a wound contact layer that may be positioned in direct contact with the wound site and the periwound area, an intermediate layer that may be positioned above the wound contact layer, and a top layer that may be positioned above the intermediate layer. The wound contact layer may comprise a plurality of apertures. Each of the layers may comprise an upper surface and a lower surface. The top layer may define a perimeter that may be configured to be positioned over the wound site and the periwound area. The intermediate layer may be positioned between the top layer and the bottom layer. The intermediate layer may have a smaller footprint than the top layer and the wound contact layer. After the period of time has passed, re-epithelialization and granulation tissue formation in the wound site may be improved compared to re-epithelialization and granulation tissue formation that would have occurred when applying negative pressure to the wound site for the period of time without the wound dressing. After the period of time has passed, erythematous tissue formation in the periwound area may be increased compared to an amount of erythematous tissue formation that would have formed when applying negative pressure to a wound for the period of time without the wound dressing.

In some embodiments, the top layer may further comprise an opening that may be configured to transmit negative pressure to the wound site and the periwound area through the wound dressing and remove bodily fluids from the wound site and the periwound area.

In some embodiments, the method disclosed may further comprise creating an opening through the top layer. The opening may be configured to transmit negative pressure to the periwound site through the wound dressing and remove bodily fluids from the wound site and the periwound area.

In some embodiments, the upper surface of the top layer may comprise one or more placement guide and the port may comprise one or more alignment features that may be configured to interact with the one or more placement guides on the upper surface of the top layer to assist a user in aligning the port with the opening on the top layer.

In some embodiments, the method for treating a wound site and a periwound area may further comprise aligning the one or more alignment features of the port with the one or more placement guides on the upper surface of the top layer.

Some of the embodiments described herein provide a system to provide negative pressure to a wound site and a periwound area surrounding the wound site. The system may comprise a wound dressing. The wound dressing may comprise a top layer and a wound contact layer. Each of the top layer and the wound contact layer can be constructed from a flexible material. Each of the top layer and the wound contact layer can comprise an upper surface and a lower surface. The top layer can define a top layer perimeter configured to be positioned over the wound site and the periwound area. The lower surface of the top layer can comprise an acrylic adhesive configured to adhere to the periwound area. The wound contact layer can be positioned below the top layer and can be configured to contact the wound site and the periwound area. The wound contact layer can comprise a plurality of apertures. The wound contact layer can define a wound contact layer perimeter. The wound contact layer perimeter can be smaller than the top layer perimeter such that portions of the top layer extend beyond the wound contact layer.

In some embodiments, the system can further comprise a port that can be configured to be secured to the upper surface of the top layer and to apply negative pressure through the top layer for the application of topical negative pressure at the wound site and the periwound area.

In some embodiments, the lower surface of the wound contact layer can comprise a silicone adhesive configured to adhere to the wound site and periwound area.

In some embodiments, the system can further comprise a first protective release layer and a second protective release layer. The first protective release layer can be configured to be removably attached to the lower surface of the portions of the top layer that extend beyond the wound contact layer. The second protective release layer can be configured to be removably attached to the lower surface of the wound contact layer.

In some embodiments, the first protective release layer can comprise a pair of protective release strips.

In some embodiments, the wound dressing can further comprise a spacer layer configured to contact the wound site and the periwound area.

In some embodiments, the spacer layer can be positioned below the wound contact layer.

Some of the embodiments described herein provide a system to provide negative pressure to a wound site and a periwound area surrounding the wound site. The system can comprise a wound dressing. The wound dressing can comprise a drape portion and a drape tail. The drape portion can comprise a top layer, a wound contact layer, and a transmission layer that can be positioned between the top layer and the wound contact layer. The drape portion can be configured to be positioned over the wound site and the periwound area. The drape tail can extend away from the drape portion. The drape tail can comprise a distal end nearer to the drape portion and a proximal end farther from the drape portion. The drape tail can comprise an elongate transmission layer extending from the distal end to the proximal end of the drape tail. The elongate transmission layer of the drape tail can be continuous with the transmission layer of the drape portion. The top layer of the drape portion can define a perimeter configured to be positioned over the wound site and the periwound area. The wound contact layer can be positioned below the top layer of the drape portion and can be configured to contact the wound site and the periwound area. The wound contact layer can comprise a plurality of apertures. The transmission layer of the drape portion can be configured to define a continuous fluid pathway from the wound site to the elongate transmission layer of the drape tail.

In some embodiments, the system can further comprise a port that can be configured to be attached to the proximal end of the drape tail and to apply negative pressure through the elongate transmission layer of the drape tail for the application of topical negative pressure at the wound site and the periwound area.

In some embodiments, the drape tail can further comprise an elongate black foam layer that can be positioned alongside the elongate transmission layer.

In some embodiments, the black foam layer can extend along an upper surface of the elongate transmission layer.

In some embodiments, the black foam layer can extend over a portion of the transmission layer of the drape portion.

In some embodiments, the drape tail can comprise a top layer extending over the elongate transmission layer from the distal end to the proximal end of the drape tail.

In some embodiments, the top layer of the drape tail can be continuous with the top layer of the drape portion.

In some embodiments, the drape tail can comprise a lower layer extending below the elongate transmission layer from the distal end to the proximal end of the drape tail.

In some embodiments, the elongate transmission layer of the drape tail and the transmission layer of the drape portion can be integrally formed from a single piece of material.

In some embodiments, the elongate transmission layer of the drape tail and the transmission layer can be formed from two separate pieces of material.

In some embodiments, the drape portion can further comprise an obscuring layer configured to obscure a presence of the wound exudate in the wound dressing.

In some embodiments, the transmission layer can be sized and configured to be positioned over the wound site and the periwound area.

In some embodiments, the system can further comprise at least one air leak in the drape portion and/or the drape tail.

In some embodiments, the system can further comprise at least one air leak at the proximal end of the drape tail.

In some embodiments, the system can further comprise a plurality of air leaks in the drape portion.

Some of the embodiments described herein provide a system to provide negative pressure to a wound site and a periwound area surrounding the wound site. The system may comprise a wound dressing. The wound dressing can comprise a top layer, an intermediate layer positioned below the top layer, a transmission layer positioned below the intermediate layer, and a wound contact layer positioned below the transmission layer. Each of the top layer, the intermediate layer, the transmission layer, and the wound contact layer can define respective perimeters. The perimeter of the top layer can be greater than the perimeter of the intermediate layer and the perimeter of the transmission layer such that portions of the top layer can extend beyond the intermediate and transmission layers. The wound contact layer can be configured to contact the wound site and the periwound area. The wound contact layer can comprise a plurality of apertures. The perimeter of the wound contact layer can be greater than the perimeter of the intermediate layer and the perimeter of the transmission layer.

In some embodiments, a lower surface of the top layer can comprise an acrylic adhesive configured to adhere to the intermediate layer and/or the wound contact layer.

In some embodiments, the acrylic adhesive can be configured to adhere to the periwound area.

In some embodiments, the system can further comprise an opening in the top layer and/or in the intermediate layer configured to supply negative pressure through the wound dressing to the wound site and to the periwound area.

In some embodiments, the system can further comprise a port configured to be secured to the upper surface of the top layer and to apply negative pressure through the top layer for the application of topical negative pressure at the wound site and the periwound area.

In some embodiments, the perimeter of the wound contact layer can be smaller than the perimeter of the top layer such that portions of the top layer can extend beyond the wound contact layer.

In some embodiments, a lower surface of the wound contact layer can comprise a silicone adhesive configured to adhere to the wound site and periwound area.

In some embodiments, the system can further comprise at least one protective release layer. The at least one protective layer can comprise a center release strip and first and second side release strips. The center release strip can be configured to be removably attached to a lower surface of the wound contact layer.

In some embodiments, the first and second side release strips can be configured to be removably attached to the lower surface of the portions of the top layer that extend beyond the wound contact layer.

In some embodiments, the first and second side release strips can be configured to be removably attached to the lower surface of wound contact layer.

In some embodiments, the center release strip, and the first and second side release strips can comprise at least one respective handle configured to remove the strips from the wound dressing when actuated.

In some embodiments, the perimeter of the intermediate layer can be greater than the perimeter of the transmission layer.

In some embodiments, the intermediate layer can be a protective construction layer configured to protect the top layer from the transmission layer.

In some embodiments, the transmission layer can comprise 3D fabric material.

In some embodiments, the intermediate layer can comprise polypropylene spunbound material.

In some embodiments, the transmission layer can be sized and positioned over the wound site and the periwound area to transmit negative pressure to the wound site and the periwound area.

Some of the embodiments described herein provide a system to provide negative pressure to a wound site and a periwound area surrounding the wound site. The system can comprise a wound dressing. The wound dressing can comprise a top layer, a spacer layer positioned below the top layer, and a wound contact layer positioned below the spacer layer. Each of the top layer, the spacer layer, and the wound contact layer can define respective perimeters. The perimeter of the top layer can be greater than the perimeter of the spacer layer such that portions of the top layer can extend beyond the spacer layer. The spacer layer can be sized to be positioned over the wound site and the periwound area to distribute negative pressure over the periwound area. The wound contact layer can be configured to contact the wound site and the periwound area. The perimeter of the wound contact layer can be greater than the perimeter of the intermediate layer.

In some configurations, the spacer layer can comprise 3D fabric material.

In some configurations, the spacer layer can comprise an acquisition distribution layer.

In some configurations, the spacer layer can be adhered directly to a lower surface of the top layer.

In some configurations, the system can further comprise an intermediate layer between the spacer layer and the top layer.

In some configurations, the intermediate layer can be a protective construction layer configured to protect the top layer from the spacer layer.

In some configurations, the intermediate layer can comprise at least one opening configured to transmit negative pressure to layers below the intermediate layer.

In some configurations, the intermediate layer can be a masking layer.

In some configurations, the intermediate layer can have a perimeter. The perimeter of the top layer can be greater than the perimeter of the intermediate layer.

In some configurations, the top layer can comprise an opening configured to transmit negative pressure to layers below the top layer.

In some configurations, the system can further comprise a port attached to the top layer configured to supply negative pressure to the wound dressing.

In some configurations, the system can further comprise one or more air leaks on the wound dressing and/or on the port.

In some configurations, the system can further comprise a drape tail extending away from the wound dressing. The drape tail can comprise a distal end nearer to the wound dressing and a proximal end farther from the wound dressing. The drape tail can comprise an elongate transmission layer extending from the distal end to the proximal end of the drape tail. The elongate transmission layer of the drape tail can be continuous with the spacer layer of the wound dressing.

In some configurations, a lower surface of the wound contact layer can comprise a silicone adhesive configured to adhere to the wound site and periwound area.

In some configurations, the system can further comprise at least one protective release layer configured to be removably attached to a lower surface of the wound contact layer.

In some configurations, an upper surface of the top layer can comprise one or more placement guides.

### BRIEF DESCRIPTION OF THE DRAWINGS

Various embodiments are depicted in the accompanying drawings for illustrative purposes, and should in no way be interpreted as limiting the scope of the embodiments. Furthermore, various features of different disclosed embodiments can be combined to form additional embodiments, which are part of this disclosure.
FIG. 1 illustrates a reduced pressure wound therapy system according to some embodiments.
FIG. 2 illustrates a pump assembly and canister according to some embodiments.
FIG. 3 illustrates an electrical component schematic of a pump assembly according to some embodiments.
FIG. 4A illustrates an embodiment of a negative pressure wound treatment system comprising a pump, and illustrating a flexible suction adapter being applied to a wound.
FIG. 4B illustrates the embodiment of FIG. 4A, with the flexible suction adapter having been placed over a wound.
FIG. 5A illustrates an isometric view of a flexible suction adapter that may be used in a negative pressure wound treatment system.
FIG. 5B illustrates an exploded view of the flexible suction adapter of FIG. 5A.
FIG. 5C illustrates a close-up view of the proximal end of the flexible suction adapter of FIG. 5B.
FIG. 5D illustrates a close-up cutaway view of the proximal end of the flexible suction adapter of FIG. 2A.
FIG. 5E illustrates a top view of the flexible suction adapter of FIG. 5A.
FIG. 5F illustrates a side view of the flexible suction adapter of FIG. 5A.
FIG. 5G illustrates a bottom view of the flexible suction adapter of FIG. 5A.
FIG. 6 illustrates an exploded view of an alternative flexible suction adapter.
FIG. 7 illustrates an exploded view of an embodiment of a drape.
FIG. 8A illustrates the placement guides on the upper surface of the top layer of the drape.
FIG. 8B illustrates the alignment features attached to the flexible suction adapter.
FIG. 9 illustrates an embodiment of the drape with the flexible suction adapter attached.
FIGS. 10A-10B illustrate an exploded view of an embodiment of a drape according to certain aspects of the present disclosure.
FIGS. 10C-10D illustrate a bottom perspective view of the embodiment of the drape shown in FIGS. 10A-10B.
FIG. 11A illustrates an exploded view of an embodiment of a drape according to certain aspects of the present disclosure.
FIG. 11B illustrates a bottom perspective view of the embodiment of the drape shown in FIG. 11A.
FIG. 12A illustrates an exploded view of an embodiment of a drape according to certain aspects of the present disclosure.
FIG. 12B illustrates a schematic of the embodiment of the drape shown in FIG. 12A.
FIG. 13A illustrates a top perspective view of a drape according to certain aspects of the present disclosure.
FIG. 13B illustrates a cross-sectional view of the drape shown in FIG. 13A.
FIG. 13C illustrates an expanded view of the drape shown in FIG. 13A.
FIG. 14 illustrates a top perspective view of a drape according to certain aspects of the present disclosure.
FIG. 15A illustrates a top perspective view of a drape according to certain aspects of the present disclosure.
FIG. 15B illustrates a cross-sectional view of the drape shown in FIG. 15A.
FIG. 15C illustrates an expanded view of the drape shown in FIG. 15A.
FIG. 16A illustrates a top perspective view of a drape according to certain aspects of the present disclosure.
FIG. 16B illustrates a cross-sectional view of the drape shown in FIG. 16A.
FIG. 16C illustrates an expanded view of the drape shown in FIG. 16A.

### DETAILED DESCRIPTION OF THE PREFERRED EMBODIMENT

Embodiments disclosed herein relate to apparatuses, systems, devices and methods of treating a wound with reduced pressure. As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (e.g., -40 mmHg is less than -60 mmHg). Negative pressure that is "more" or "greater" than -X mmHg corresponds to pressure that is further from atmospheric pressure (e.g., -80 mmHg is more than -60 mmHg). In some embodiments, local ambient atmospheric pressure is used as a reference point, and such local atmospheric pressure may not necessarily be, for example, 760 mmHg.

Embodiments of the present disclosure are generally applicable to use in topical negative pressure (TNP) or reduced pressure therapy systems. Briefly, negative pressure wound therapy assists in the closure and healing of many forms of "hard to heal" wounds by reducing tissue oedema, encouraging blood flow and granular tissue formation, or removing excess exudate and can reduce bacterial load (and thus infection risk). In addition, the therapy allows for less disturbance of a wound leading to more rapid healing. TNP therapy systems can also assist in the healing of surgically closed wounds by removing fluid. In some embodiments, TNP therapy helps to stabilize the tissue in the apposed position of closure. A further beneficial use of TNP therapy can be found in grafts and flaps where removal of excess fluid is important and close proximity of the graft to tissue is required in order to ensure tissue viability.

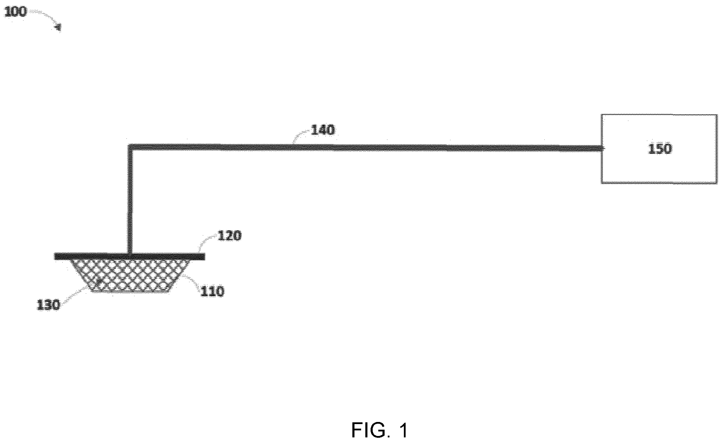

Embodiments described herein relate to materials, apparatuses, methods, and systems that incorporate, or comprise, or utilize a wound contact layer ("WCL") for positioning in contact with a wound. A WCL may be utilized as a stand-alone component for separately positioning at a wound site, or may be incorporated into any number of multi-layer wound dressings and wound treatment apparatuses, such as described herein below with respect to Figs. 1 through 9. Embodiments of the present disclosure are generally applicable to use under ambient conditions, in negative pressure or reduced pressure therapy systems, or in compression therapy systems.

Some of the preferred embodiments described herein incorporate, or comprise, or utilize multi-care wound contact layers. Such a multi-care WCL possesses two or more of the following functional features: antimicrobial activities, easiness to apply or/and remove as one piece, easiness to cut with scissors, conformability to the three-dimensional contour of a wound surface, durability to wear, compatibility with negative pressure wound therapy or/and compression wound therapy, exudate management, capability of facilitating autolytic debridement of wound, capability of promoting wound healing, and self-indication of compositional or functional changes.

Certain embodiments described herein provide a wound treatment system. Such a wound treatment system may comprise a stand-alone layer of multi-care WCL, configured to be sized for positioning over a wound. The wound treatment system may further comprise a secondary wound dressing configured to be separately positioned over the multi-care WCL. The multi-care WCL may have an adhesive adhered to the lower surface; and the adhesive can be configured that the multi-care WCL will be placed in proximity to the wound. The secondary wound dressing, if used, may adhere to skin surrounding the wound and may have the same size or may be larger than the multi-care WCL, so that the multi-care WCL will touch or be placed in proximity to the wound. The secondary wound dressing can be alternatively or additionally configured to form a seal to skin surrounding the wound so that the multi-care WCL will touch or be placed in proximity to the wound. The wound treatment system may further comprise a source of negative pressure configured to supply negative pressure through the secondary wound dressing and through the multi-care wound contact layer to the wound.

Certain other embodiments described herein provide a multi-layered wound dressing, such as described herein the specification with respect to Figs. 1 through 9. Such a multi-layered wound dressing may incorporate a multi-care WCL as a component layer thereof or, alternatively, may comprise a composite or laminate including the multi-care WCL as part of one of the component layers thereof. The multi-layered wound dressing may comprise: a multi-care wound contact layer as described above or described elsewhere herein; a transmission layer and/or absorbent layer over the multi-care wound contact layer; and a cover layer over the transmission layer and/or absorbent layer. The wound dressing may further comprise an adhesive layer on the lower surface of the multi-care wound contact layer. The wound dressing may further comprise a negative pressure port positioned on or above the cover layer. The multi-care wound contact layer may have a perimeter shape that is substantially the same as a perimeter shape of the cover layer. Alternatively, the multi-care wound contact layer may have a perimeter shape that is smaller than a perimeter shape of the cover layer.

Some embodiments described herein the specification provide a method to treat a wound or locus. Such a method may include placing a multi-care WCL, either separately or by placing a multi-layered wound dressing having a multi-care WCL, over the wound. The method may comprise adhering the separate multi-care WCL and/or the multi-layer wound dressing having a multi-care WCL to healthy skin around the wound. The method may further comprise one or more of the following steps: A further wound dressing can be placed over the separate multi-care WCL or multi-layered wound dressing having the multi-care WCL that is placed over the wound. Wound exudate, or any moist or aqueous medium other than wound exudate, may be provided to reach and/or touch the multi-care WCL. Wound exudate, or any moist or aqueous medium other than wound exudate may be diffused or wicked into the wound dressing incorporating the multi-care WCL or into a wound dressing provided over the multi-care WCL. Negative pressure may be applied to the separate multi-care WCL or multi-layered wound dressing having the multi-care WCL, such that wound exudate is suctioned into the multi-care WCL directly, or into the wound dressing incorporating the multi-care WCL, or into a wound dressing provided over the multi-care WCL.

### Negative Pressure System

Fig. 1 illustrates an embodiment of a negative or reduced pressure wound treatment (or TNP) system 100 including a wound filler 130 placed inside a wound cavity 110, the wound cavity sealed by a wound cover 120. The wound filler 130 in combination with the wound cover 120 can be referred to as wound dressing. A flow path 140, such as a single or multi lumen tube or conduit, is connected to the wound cover 120 with a negative pressure wound therapy device, for example pump assembly 150, configured to supply reduced pressure. The wound cover 120 can be in fluidic communication with the wound cavity 110. In any of the system embodiments disclosed herein, as in the embodiment illustrated in Fig. 1, the pump assembly can be a canisterless pump assembly (meaning that exudate is collected in the wound dressing or is transferred via tube 140 for collection to another location). However, any of the pump assembly embodiments disclosed herein can be configured to include or support a canister. Additionally, in any of the system embodiments disclosed herein, any of the pump assembly embodiments can be mounted to or supported by the dressing, or adjacent to the dressing. The wound filler 130 can be any suitable type, such as hydrophilic or hydrophobic foam, gauze, inflatable bag, and so on. The wound filler 130 can be conformable to the wound cavity 110 such that it substantially fills the cavity. The wound cover 120 can provide a substantially fluid impermeable seal over the wound cavity 110. The wound cover 120 can have a top side and a bottom side, and the bottom side adhesively (or in any other suitable manner) seals with wound cavity 110. The conduit 140 or lumen or any other conduit or lumen disclosed herein can be formed from polyurethane, PVC, nylon, polyethylene, silicone, or any other suitable material.

Some embodiments of the wound cover 120 can have a port (not shown) configured to receive an end of the conduit 140. In other embodiments, the conduit 140 can otherwise pass through or under the wound cover 120 to supply reduced pressure to the wound cavity 110 so as to maintain a desired level of reduced pressure in the wound cavity. The conduit 140 can be any suitable article configured to provide at least a substantially sealed fluid flow pathway between the pump assembly 150 and the wound cover 120, so as to supply the reduced pressure provided by the pump assembly 150 to wound cavity 110. The wound cover 120 and the wound filler 130 can be provided as a single article or an integrated single unit. In some embodiments, no wound filler is provided and the wound cover by itself may be considered the wound dressing. The wound dressing may then be connected, via the conduit 140, to a source of negative pressure, such as the pump assembly 150. The pump assembly 150 can be miniaturized and portable, although larger conventional pumps such can also be used.

The wound cover 120 can be located over a wound site to be treated. The wound cover 120 can form a substantially sealed cavity or enclosure over the wound site. In some embodiments, the wound cover 120 can be configured to have a film having a high water vapour permeability to enable the evaporation of surplus fluid, and can have a superabsorbing material contained therein to safely absorb wound exudate. It will be appreciated that throughout this specification reference is made to a wound. In this sense it is to be understood that the term wound is to be broadly construed and encompasses open and closed wounds in which skin is torn, cut or punctured or where trauma causes a contusion, or any other surficial or other conditions or imperfections on the skin of a patient or otherwise that benefit from reduced pressure treatment. A wound is thus broadly defined as any damaged region of tissue where fluid may or may not be produced. Examples of such wounds include, but are not limited to, acute wounds, chronic wounds, surgical incisions and other incisions, subacute and dehisced wounds, traumatic wounds, flaps and skin grafts, lacerations, abrasions, contusions, burns, diabetic ulcers, pressure ulcers, stoma, surgical wounds, trauma and venous ulcers or the like. The components of the TNP system described herein can be particularly suited for incisional wounds that exude a small amount of wound exudate.

Some embodiments of the system are designed to operate without the use of an exudate canister. Some embodiments can be configured to support an exudate canister. In some embodiments, configuring the pump assembly 150 and tubing 140 so that the tubing 140 can be quickly and easily removed from the pump assembly 150 can facilitate or improve the process of dressing or pump changes, if necessary. Any of the pump embodiments disclosed herein can be configured to have any suitable connection between the tubing and the pump.

In some embodiments, the pump assembly 150 can be configured to deliver negative pressure of approximately -80 mmHg, or between about -20 mmHg and -200 mmHg. Note that these pressures are relative to normal ambient atmospheric pressure thus, -200 mmHg would be about 560 mmHg in practical terms. The pressure range can be between about -40 mmHg and -150 mmHg. Alternatively a pressure range of up to -75 mmHg, up to -80 mmHg or over -80 mmHg can be used. Also a pressure range of below -75 mmHg can be used. Alternatively a pressure range of over approximately -100 mmHg, or even 150 mmHg, can be supplied by the pump assembly 150. In some embodiments, the pump assembly 150 is configured to provide continuous or intermittent negative pressure therapy. Continuous therapy can be delivered at above -25 mmHg, -25 mmHg, -40 mmHg, -50 mmHg, -60 mmHg, -70 mmHg, -80 mmHg, -90 mmHg, -100 mmHg, -120 mmHg, -140 mmHg, -160 mmHg, -180 mmHg, -200 mmHg, or below -200 mmHg. Intermittent therapy can be delivered between low and high negative pressure setpoints. Low setpoint can be set at above 0 mmHg, 0 mmHg, -25 mmHg, -40 mmHg, -50 mmHg, -60 mmHg, -70 mmHg, -80 mmHg, -90 mmHg, -100 mmHg, -120 mmHg, -140 mmHg, -160 mmHg, -180 mmHg, or below -180 mmHg. High setpoint can be set at above -25 mmHg, -40 mmHg, -50 mmHg, -60 mmHg, -70 mmHg, -80 mmHg, -90 mmHg, -100 mmHg, -120 mmHg, -140 mmHg, -160 mmHg, -180 mmHg, -200 mmHg, or below -200 mmHg. During intermittent therapy, negative pressure at low setpoint can be delivered for a first-time duration, and upon expiration of the first-time duration, negative pressure at high setpoint can be delivered for a second-time duration. Upon expiration of the second-time duration, negative pressure at low setpoint can be delivered. The first and second time durations can be same or different values. The first and second durations can be selected from the following range: less than 2 minutes, 2 minutes, 3 minutes, 4 minutes, 6 minutes, 8 minutes, 10 minutes, or greater than 10 minutes. In some embodiments, switching between low and high setpoints and vice versa can be performed according to a step waveform, square waveform, sinusoidal waveform, and the like.

In some embodiments, the TNP system 100 can include multiple wound dressings connected to the pump assembly 150. The performance and wound healing capabilities (such as, fluid management) of the TNP system with multiple wound dressings with the pump assembly 150 can be equivalent to or exceed that of a standard single wound dressing with single pump set-up.

In operation, the wound filler 130 is inserted into the wound cavity 110 and wound cover 120 is placed so as to seal the wound cavity 110. The pump assembly 150 provides a source of a negative pressure to the wound cover 120, which is transmitted to the wound cavity 110 via the wound filler 130. Fluid (e.g., wound exudate) is drawn through the conduit 140, and can be stored in a canister. In some embodiments, fluid is absorbed by the wound filler 130 or one or more absorbent layers (not shown).

Wound dressings that may be utilized with the pump assembly and other embodiments of the present application include Renasys-F, Renasys-G, Renasys AB, and Pico Dressings available from Smith & Nephew. Any of the dressings described herein can be used with Smith and Nephew's Renasys Soft Port connector or interface between the dressing and the pump assembly. For example, the Renasys Soft Port connector can be positioned in the flow path 140 and serve as a port for the wound dressing. In other embodiments, other suitable wound dressings can be utilized.

### Pump Assembly and Canister

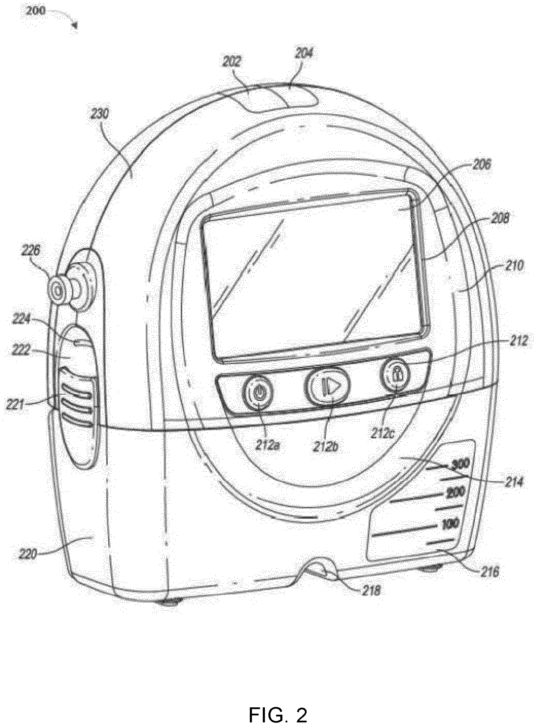

Fig. 2 illustrates a front view 200 of a pump assembly 230 and canister 220 according to some embodiments. As is illustrated, the pump assembly 230 and the canister are connected, thereby forming a TNP device or system. The pump assembly 230 includes one or more indicators, such as visual indicator 202 configured to indicate alarms and visual indicator 204 configured to indicate status of the TNP system. The indicators 202 and 204 can be configured to alert a user, such as patient or medical care provider, to a variety of operating or failure conditions of the system, including alerting the user to normal or proper operating conditions, pump failure, power supplied to the pump or power failure, detection of a leak within the wound cover or flow pathway, suction blockage, no flow condition, canister full condition, or any other similar or suitable conditions or combinations thereof. The pump assembly 230 can include additional indicators. The pump assembly can use a single indicator or multiple indicators. Any suitable indicator can be used such as visual, audio, tactile indicator, and so on. The indicator 202 can be configured to signal alarm conditions, such as canister full, power low, conduit 140 disconnected, seal broken in the wound seal 120, and so on. The indicator 202 can be configured to display red flashing light to draw user's attention. The indicator 204 can be configured to signal status of the TNP system, such as therapy delivery is ok, leak detected, and so on. The indicator 204 can be configured to display one or more different colors of light, such as green, yellow, etc. For example, green light can be emitted when the TNP system is operating properly and yellow light can be emitted to indicate a warning.

The pump assembly 230 may include a display or screen 206 mounted in a recess 208 formed in a case of the pump assembly. The display 206 can be a touch screen display. The display 206 can support playback of audiovisual (AV) content, such as instructional videos. As explained herein, the display 206 can be configured to render a number of screens or graphical user interfaces (GUIs) for configuring, controlling, and monitoring the operation of the TNP system. The pump assembly 230 includes a gripping portion 210 formed in the case of the pump assembly. The gripping portion 210 can be configured to assist the user to hold the pump assembly 230, such as during removal of the canister 220. The canister 220 can be replaced with another canister, such as when the canister 220 has been filled with fluid.

The pump assembly 230 may include one or more keys or buttons 212 configured to allow the user to operate and monitor the operation of the TNP system. As is illustrated, there buttons 212a, 212b, and 212c are included. Button 212a can be configured as a power button to turn on/off the pump assembly 230. Button 212b can be configured as a play/pause button for the delivery of negative pressure therapy. For example, pressing the button 212b can cause therapy to start, and pressing the button 212b afterward can cause therapy to pause or end. Button 212c can be configured to lock the display 206 or the buttons 212. For instance, button 212c can be pressed so that the user does not unintentionally alter the delivery of the therapy. Button 212c can be depressed to unlock the controls. In other embodiments, additional buttons can be used or one or more of the illustrated buttons 212a, 212b, or 212c can be omitted. Multiple key presses or sequences of key presses can be used to operate the pump assembly 230.

The pump assembly 230 may include one or more latch recesses 222 formed in the cover. In the illustrated embodiment, two latch recesses 222 can be formed on the sides of the pump assembly 230. The latch recesses 222 can be configured to allow attachment and detachment of the canister 220 using one or more canister latches 221. The pump assembly 230 includes an air outlet 224 for allowing air removed from the wound cavity 110 to escape. Air entering the pump assembly can be passed through one or more suitable filters, such as antibacterial filters. This can maintain reusability of the pump assembly. The pump assembly 230 includes one or more strap mounts 226 for connecting a carry strap to the pump assembly 230 or for attaching a cradle. In the illustrated embodiment, two strap mounts 226 can be formed on the sides of the pump assembly 230. In some embodiments, various features are omitted or various additional features are added to the pump assembly 230.

The canister 220 may be configured to hold fluid (e.g., exudate) removed from the wound cavity 110. The canister 220 includes one or more latches 221 for attaching the canister to the pump assembly 230. In the illustrated embodiment, the canister 220 includes two latches 221 on the sides of the canister. The exterior of the canister 220 can formed from frosted plastic so that the canister is substantially opaque and the contents of the canister and substantially hidden from plain view. The canister 220 includes a gripping portion 214 formed in a case of the canister. The gripping portion 214 can be configured to allow the user to hold the pump assembly 220, such as during removal of the canister from the apparatus 230. The canister 220 includes a substantially transparent window 216, which can also include graduations of volume. For example, the illustrated 300 mL canister 220 includes graduations of 50 mL, 100 mL, 150 mL, 200 mL, 250 mL, and 300 mL. Other embodiments of the canister can hold different volume of fluid and can include different graduation scale. For example, the canister can be an 800 mL canister. The canister 220 includes a tubing channel 218 for connecting to the conduit 140. In some embodiments, one or more of these features, such as the gripping portion 214, are omitted or various additional features are added to the canister 220. Any of the disclosed canisters may include or may omit a solidifier.

### Electronics and Software

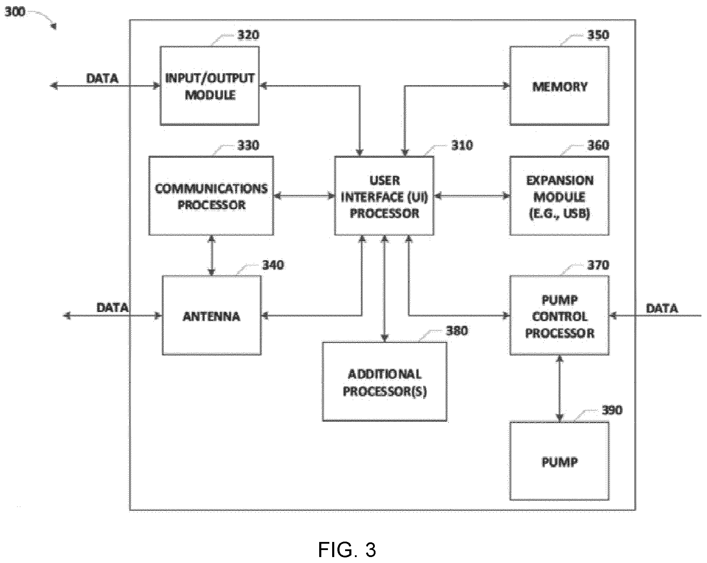

Fig. 3 illustrates an electrical component schematic 300 of a pump assembly, such as the pump assembly 230, according to some embodiments. Electrical components can operate to accept user input, provide output to the user, operate the pump assembly and the TNP system, provide network connectivity, and so on. Electrical components can be mounted on one or more printed circuit boards (PCBs). As is illustrated, the pump assembly can include multiple processors. It may be advantageous to utilize multiple processors in order to allocate or assign various tasks to different processors. A first processor can be responsible for user activity and a second processor can be responsible for controlling the pump. This way, the activity of controlling the pump, which may necessitate a higher level of responsiveness (corresponding to higher risk level), can be offloaded to a dedicated processor and, thereby, will not be interrupted by user interface tasks, which may take longer to complete because of interactions with the user.

The pump assembly can include a user interface processor or controller 310 configured to operate one or more components for accepting user input and providing output to the user, such as the display 206, buttons 212, etc. Input to the pump assembly and output from the pump assembly can controlled by an input/output (I/O) module 320. For example, the I/O module can receive data from one or more ports, such as serial, parallel, hybrid ports, and the like. The processor 310 also receives data from and provides data to one or more expansion modules 360, such as one or more USB ports, SD ports, Compact Disc (CD) drives, DVD drives, FireWire ports, Thunderbolt ports, PCI Express ports, and the like. The processor 310, along with other controllers or processors, stores data in one or more memory modules 350, which can be internal or external to the processor 310. Any suitable type of memory can be used, including volatile or non-volatile memory, such as RAM, ROM, magnetic memory, solid-state memory, Magnetoresistive random-access memory (MRAM), and the like.

In some embodiments, the processor 310 can be a general-purpose controller, such as a low-power processor. In other embodiments, the processor 310 can be an application specific processor. The processor 310 can be configured as a "central" processor in the electronic architecture of the pump assembly, and the processor 310 can coordinate the activity of other processors, such as a pump control processor 370, communications processor 330, and one or more additional processors 380 (e.g., processor for controlling the display 206, processor for controlling the buttons 212, etc.). The processor 310 can run a suitable operating system, such as a Linux, Windows CE, VxWorks, etc.

The pump control processor 370 can be configured to control the operation of a negative pressure source or pump 390. The pump 390 can be a suitable pump, such as a diaphragm pump, peristaltic pump, rotary pump, rotary vane pump, scroll pump, screw pump, liquid ring pump, pump (for example, diaphragm pump) operated by a piezoelectric transducer, voice coil pump, and the like. The pump control processor 370 can measure pressure in a fluid flow path, using data received from one or more pressure sensors, calculate the rate of fluid flow, and control the pump. The pump control processor 370 can control an actuator, such as a pump motor, so that a desired level of negative pressure is achieved in the wound cavity 110. The desired level of negative pressure can be pressure set or selected by the user. In various embodiments, the pump control processor 370 controls the pump actuator (e.g., pump motor) using pulse-width modulation (PWM). A control signal for driving the pump actuator can be a 0-100% duty cycle PWM signal. The pump control processor 370 can perform flow rate calculations and detect various conditions in a flow path. The pump control processor 370 can communicate information to the processor 310. The pump control processor 370 can include internal memory or can utilize memory 350. The pump control processor 370 can be a low-power processor.

A communications processor 330 can be configured to provide wired or wireless connectivity. The communications processor 330 can utilize one or more antennas 340 for sending and receiving data. The communications processor 330 can provide one or more of the following types of connections: Global Positioning System (GPS) technology, cellular connectivity (e.g., 2G, 3G, LTE, 4G), Wi-Fi connectivity, Internet connectivity, and the like. Connectivity can be used for various activities, such as pump assembly location tracking, asset tracking, compliance monitoring, remote selection, uploading of logs, alarms, and other operational data, and adjustment of therapy settings, upgrading of software or firmware, and the like. The communications processor 330 can provide dual GPS/cellular functionality. Cellular functionality can, for example, be 3G functionality. In such cases, if the GPS module is not able to establish satellite connection due to various factors including atmospheric conditions, building or terrain interference, satellite geometry, and so on, the device location can be determined using the 3G network connection, such as by using cell identification, triangulation, forward link timing, and the like. The pump assembly can include a SIM card, and SIM-based positional information can be obtained.

The communications processor 330 can communicate information to the processor 310. The communications processor 330 can include internal memory or can utilize memory 350. The communications processor 330 can be a low-power processor.

In some embodiments, the pump assembly can track and store various data, such as one or more of positioning data, therapy parameters, logs, device data, and so on. The pump assembly can track and log therapy and other operational data. Data can be stored, for example, in the memory 350.

In some embodiments, using the connectivity provided by the communications processor 330, the device can upload any of the data stored, maintained, or tracked by the pump assembly. For example, the following information can be uploaded to a remote computer or server: activity log(s), which includes therapy delivery information, such as therapy duration, alarm log(s), which includes alarm type and time of occurrence; error log, which includes internal error information, transmission errors, and the like; therapy duration information, which can be computed hourly, daily, and the like; total therapy time, which includes therapy duration from first applying a particular therapy program or programs; lifetime therapy information; device information, such as the serial number, software version, battery level, etc.; device location information; patient information; and so on. The device can also download various operational data, such as therapy selection and parameters, firmware and software patches and upgrades, and the like. The pump assembly can provide Internet browsing functionality using one or more browser programs, mail programs, application software (e.g., apps), etc.

In some embodiments, the communications processor 330 can use the antenna 340 to communicate a location of the pump assembly, such as a location of a housing of the pump assembly, to other devices in the proximity (for example, within 10, 20, or 50 meters and the like) of the pump assembly. The communications processor 330 can perform one-way or two-way communication with the other devices depending on the implementation. The communications transmitted by the communications processor 330 can include identifying information to uniquely identify the pump assembly relative to one or more other pump assemblies also in the proximity of the pump assembly. For example, identifying information can include a serial number or a value derived from the serial number. The signal strength of the transmitted communications by the communications processor 330 can be controlled (for example, maintained at a constant or substantially constant level) to enable another device to determine a distance to the pump assembly, such as a distance between the device and the pump assembly.

In some embodiments, the communications processor 330 can communicate with other devices in the proximity of the pump assembly so that the communications processor 330 can itself determine a distance from the pump assembly to the other devices. The communications processor 330, in such embodiments, can track and store the distance from the pump assembly to the other devices or indications of change in the distance over time, and the communications processor 330 can later provide this information to the other devices. For instance, the communications processor 330 can determine a duration of time during which the pump assembly has been removed from a coverage area of a device and subsequently report this time to the device upon being returned to the coverage area.

### Flexible Suction Adapter

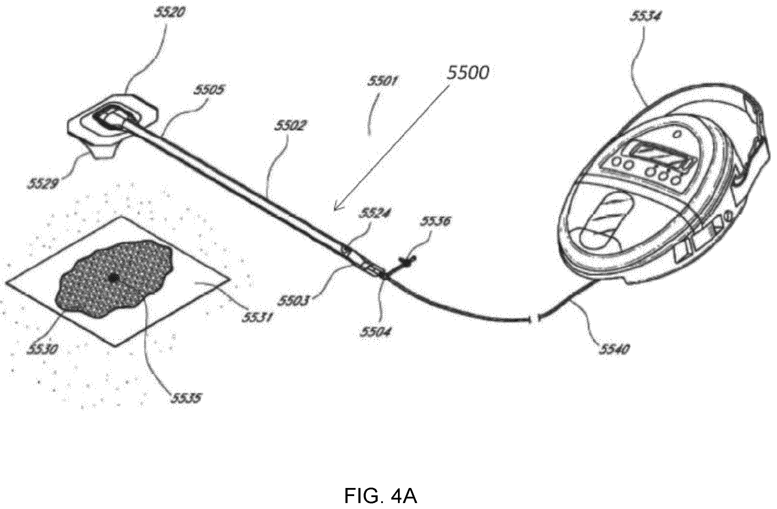

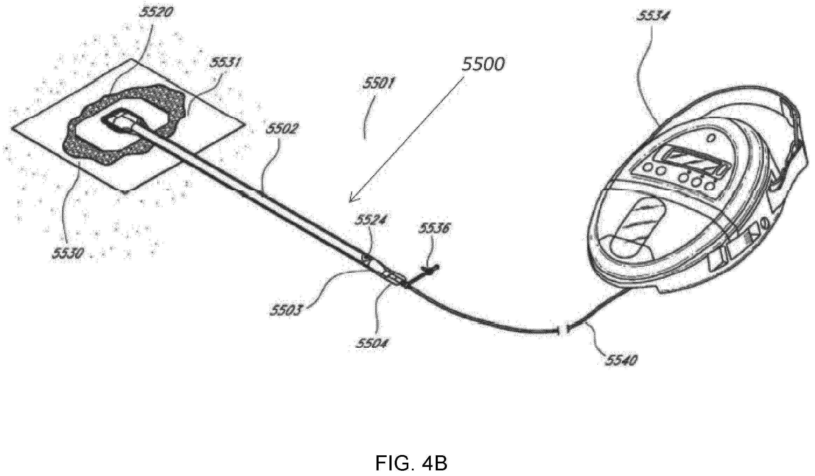

Figs. 4A-6 illustrate embodiments of a negative pressure wound treatment system 5501 similar to the embodiment illustrated in Fig. 1. Here, the system 5501 may comprise a port in the form of a flexible suction adapter 5500 having a bridge portion 5502 with a proximal end 5503 and a distal end 5505, and an applicator 5520 at the distal end 5505 of the bridge portion 5502 forming the flexible suction adapter 5500. A connector 5504 is preferably disposed at the proximal end 5503 of the bridge portion 5502, so as to connect to at least one of the channels 5512 and/or 5516, as shown in Fig. 4B. A cap 5536 may be provided with the system 5501 (and can in some cases, as illustrated, be attached to the connector 5504). The cap 5536 can be useful in preventing fluids from leaking out of the proximal end 5503. The system 5501 may include a source of negative pressure such as a pump or negative pressure unit 5534 capable of supplying negative pressure. The pump also preferably comprises a canister or other container for the storage of wound exudates and other fluids that may be removed from the wound. In some embodiments, this pump 5534 may be the pump 200 described in relation to Fig. 2. In some embodiments, this pump 5534 can be a RENASYS GO pump, as sold by Smith & Nephew. The pump 5534 may be connected to the connector 5504 via a tube 5540. In use, the applicator 5520 is placed over an aperture 5535 formed in a drape 5531 that is placed over a suitably-prepared wound 5530, which may in some cases be filled with a wound packing material such as foam or gauze. Subsequently, with the pump 5534 connected via the tube 5540 to the connector 5504, the pump is activated, thereby supplying negative pressure to the wound. Application of negative pressure may be applied until a desired level of healing of the wound 5530 is achieved.

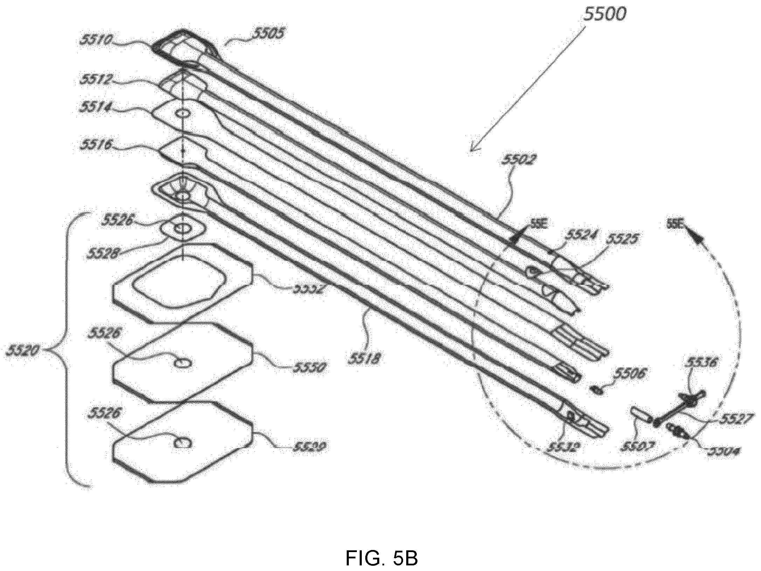

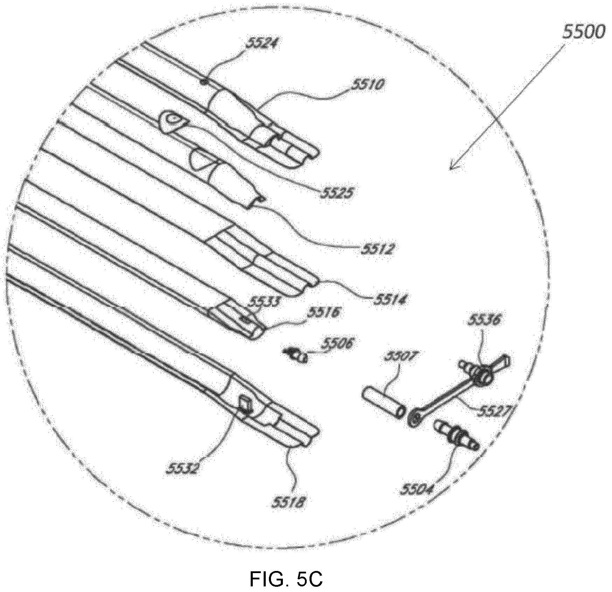

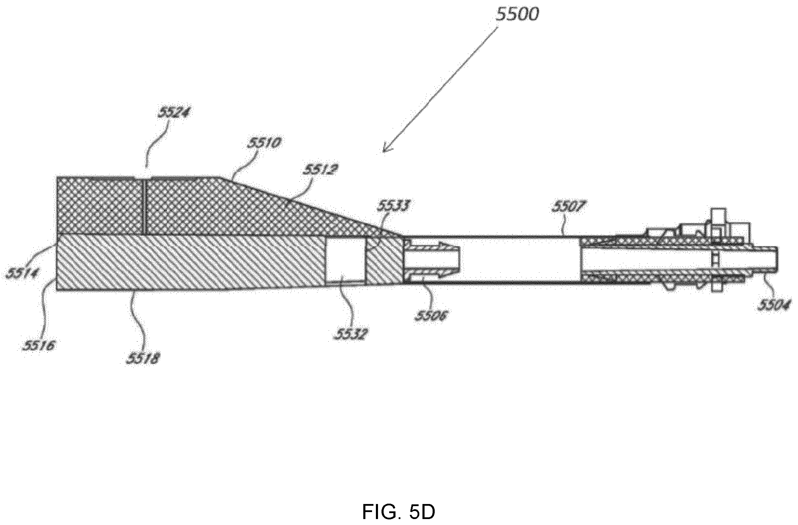

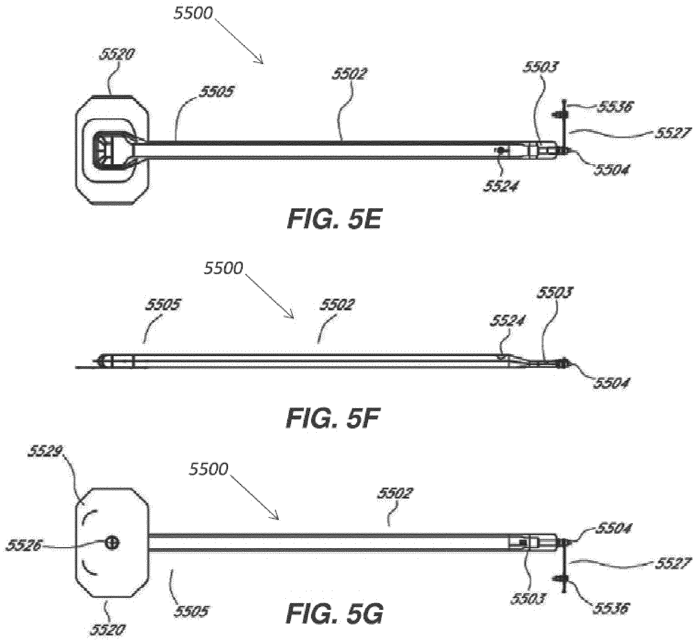

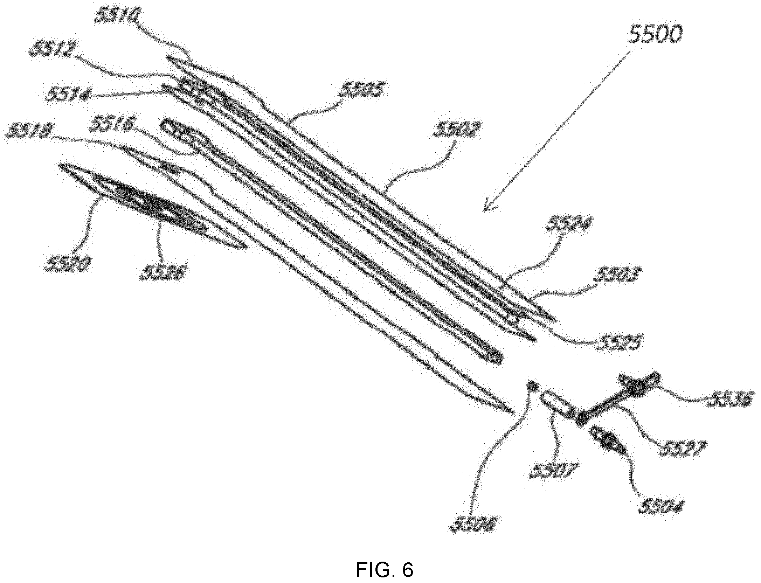

In some embodiments, the bridge portion 5502 may comprise an upper channel layer 5512 positioned between an upper layer 5510 and an intermediate layer 5514, with a lower channel layer 5516 positioned between the intermediate layer 5514 and a bottom layer 5518. Preferably, the layers 5510, 5514, and 5518 have elongate portions extending between proximal and distal ends and may be comprised of a material that is fluid-impermeable, for example polymers such as polyurethane. It will of course be appreciated that the layers 5510, 5514, and 5518 may each be constructed from different materials, including semi-permeable materials. In some embodiments, one or more of the layers 5510, 5514, and 5518 may be at least partially transparent. As illustrated in FIG. 5B, the upper and lower layers 5510 and 5518 may be curved, rounded or outwardly convex over a majority of their lengths. During assembly, for example, the layers 5510, 5514, and 5518 may be pinched together to weld or adhere the layers together. In doing so, the proximal ends of the channels 5512 and 5516 may be sandwiched between these layers, thus partially compressing the proximal ends of the channels 5512, 5516 and stretching the layers 5510, 5514, 5518 over these aforementioned proximal ends. Of course, the proximal ends of the materials used in the bridge portion 5502 may not necessarily be rounded or curved; as shown in Fig. 6, they can remain substantially squared off and straight.

The upper and lower channel layers 5512 and 5516 are preferably elongate layers extending from the proximal end 5503 to the distal end 5505 and may each preferably comprise a porous material, including for example open-celled foams such as polyethylene or polyurethane. In some embodiments, one or more of the upper and lower channel layers 5512 and 5516 may be comprised of a fabric, for example a knitted or woven spacer fabric (such as a knitted polyester 3D fabric, Baltex 7970.RTM., or Gehring 879.RTM.) or a nonwoven material. Suitable materials may also include terry-woven or loop-pile materials. The fibers may not necessarily be woven, and can include felted and flocked (including materials such as Flotex.RTM.) fibrous materials. The materials selected are preferably suited to channeling wound exudate away from the wound and for transmitting negative pressure and/or vented air to the wound site, and may also confer a degree of kinking or occlusion resistance to the channel layers 5512 and 5516 as described below. In one embodiment, the upper channel layer 5512 may comprise an open-celled foam such as polyurethane, and the lower channel layer may comprise a fabric as described herein. In another embodiment, the upper channel layer is optional, and the system may instead be provided with an open upper channel. In the embodiment illustrated in Fig. 5B, the upper channel layer 5512 may have a curved, rounded or upwardly convex upper surface and a substantially flat lower surface, and the lower channel layer 5516 may have a curved, rounded or downwardly convex lower surface and a substantially flat upper surface.

In some embodiments, the fabric may have a three-dimensional (3D) structure, where one or more types of fibers form a structure where the fibers extend in all three dimensions. Such a fabric may in some cases aid in wicking, transporting fluid, and/or transmitting negative pressure. To prevent the channels 5512 and/or 5516 from being displaced or twisted while encased in the system 5501--which may impair performance of the respective channels under negative pressure--it may in some embodiments be preferable to adhere or otherwise secure the channels 5512 and/or 5516 to one or more of the layers 5510, 5514, and 5518. In certain embodiments, these materials remain open and capable of communicating negative pressure to a wound area under the typical pressures used in negative pressure therapy, for example between 40 to 150 mmHg, although higher and lower values are possible. In some embodiments, the fabric may comprise several layers of material stacked or layered over each other, which may in some cases be useful in preventing the channel 5516 from collapsing under the application of negative pressure. In other embodiments, the fabric used in channel 5516 may be between 1.5 mm and 6 mm; more preferably, the fabric may be between 3 mm and 6 mm thick, and may be comprised of either one or several individual layers of fabric. In other embodiments, the channel 5512 may be between 1.2-3 mm thick, and preferably thicker than 1.5 mm. Additionally, and as described previously, the materials used in the system 5501 are preferably conformable and soft, which may help to avoid pressure ulcers and other complications which may result from a wound treatment system being pressed against the skin of a patient.

In some embodiments, the distal ends of the layers 5510, 5514, and 5518 and the channel layers 5512 and 5516 are enlarged at their distal ends (to be placed over a wound site), and may form a "teardrop" or other enlarged shape. The distal ends of at least the layers 5512, 5514, 5516, and 5518 may also be provided with at least one through aperture. This aperture may be useful not only for the drainage of wound exudate and for applying negative pressure to the wound, but also during manufacturing of the device, as these apertures may be used to align these respective layers appropriately.

With additional reference to Figs. 5B-C and 6, a channel connector 5506 may be provided at the proximal end 5503 of the bridge portion 5502, the channel connector 5506 preferably being configured so as to be embedded into the lower channel layer 5516 so as to create a secure fluidic connection. The channel connector 5506 may in some embodiments be inserted into a pre-made cavity formed into the channel 5516; as illustrated in Fig. 6, this cavity can be cut out or can be in the form of a rabbet joint. With one end of the channel connector 5506 being embedded into the lower channel layer 5516, the other end of the channel connector 5506 may be connected or in communication with, in one embodiment, a connector tube 5507, although in some embodiments the channel connector 5506 may be connected directly to the connector 5504, or else connected directly to a tube 5540 connected to a source of negative pressure. When using a connector tube 5507, the resulting assembly can permit a connector 5504 to be attached thereto. A cap 5536, which may be secured to the suction adapter for example via a cap leash 5527 secured with a ring disposed on the outer surface of the connector tube 5507. The cap 5536 may be used to cover the end of the suction adapter, for example at the connector 5504, so as to prevent exudate and other wound fluids from leaking out. The connector 5504 is preferably configured to connect with a tube 5540 connected to a source of negative pressure. The connector 5504 may for example comprise a lip or other such structure to aid in securing the connector 5504 to a tube 5540 and/or cap 5536, although it will be understood that other connector types are possible, including quick-disconnect couplings, luer locks, Christmas-tree, and other such connectors.

The upper layer 5510 may comprise additional material extending downward, preferably at least of the thickness of the bridge portion 5502; this material may then be used to bond or weld to the other layers so to form a fluid-tight seal. More specifically, during assembly, the upper layer 5510 may be attached, for example by melting, welding, or with adhesives, to the lower layer 5518 so as to form a fluid-tight seal (with the exception of the apertures at the distal and proximal ends). Preferably, the middle layer 5514 is attached to the top layer 5510 and the bottom layer 5518. In some embodiments, it may be preferable to attach or bond the connectors 5504 and/or 5506, as well as the tube 5507 to at least one of the layers 5510, 5514, 5518 so as to create a fluid-tight connection. To provide for a more secure connection, some embodiments may also be provided with a weld 5532 made onto the lower layer 5518. The lower channel 5516 may have a hole or aperture made through it, which may be used to weld it, via the weld 5532, to the lower layer 5518. This welding of the lower channel 5516 to the lower layer 5518 via the weld 5532 made through the hole 5533 may thus aid in preventing the various layers and channels from shifting or being displaced. Obviously, it will be understood that other securement means may be used, for example adhesives and the like, and that such arrangements may be also be used in the upper channel 5512.

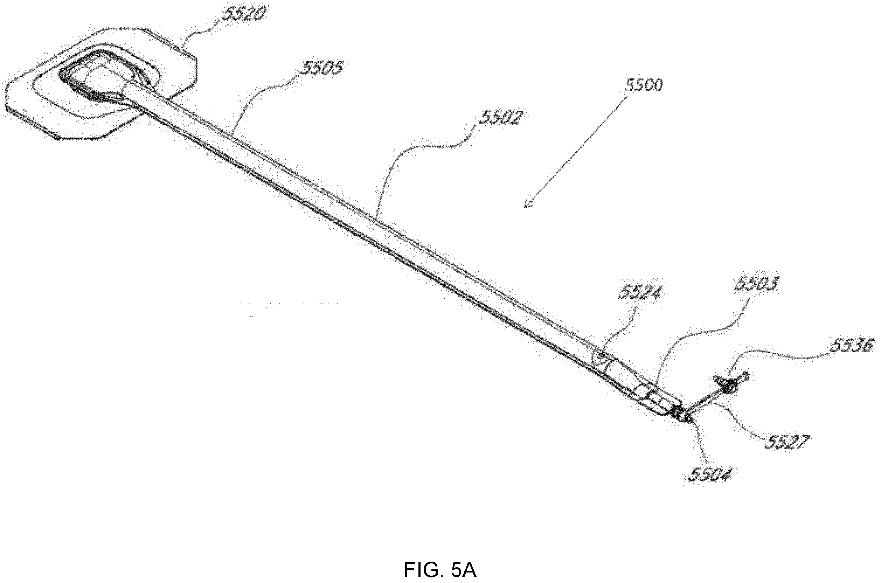

**In** certain embodiments, for example as illustrated in Figs. 5A-6, a controlled air leak 5524 may be disposed on the bridge portion 5502, for example at the proximal end thereof. This air leak 5524 may comprise an opening or channel extending through upper layer 5510, such that the air leak 5524 is in fluidic communication with the upper channel 5512. Upon the application of suction to the suction adapter 5500, air will enter through the air leak 5524 and move from the proximal end 5503 to the distal end 5505 along the upper channel 5512. The air will then be suctioned into the lower channel 5516 by passing through the apertures through the distal ends of the layers 5512, 5514, 5516 and 5518. The air leak 5524 preferably comprises a filter 5525. Preferably, the air leak 5524 is located at the proximal end of the bridge portion 5502 so as to minimize the likelihood of wound exudate or other fluids coming into contact and possibly occluding or interfering with the air leak 5524 or its filter 5525. In some embodiments, this filter 5525 is a microporous membrane capable of excluding microorganisms and bacteria, and which may be able to filter out particles larger than 45 µm. Preferably, the filter 5525 can exclude particles larger than 1.0 µm, and more preferably, particles larger than 0.2 µm. Advantageously, some embodiments may provide for a filter 5525 that is at least partially chemically-resistant, for example to water, common household liquids such as shampoos, and other surfactants. In some embodiments, reapplication of vacuum to the suction adapter 5500 and/or wiping of the exposed outer portion of the filter 5525 may be sufficient to clear any foreign substance occluding the filter 5525. The filter 5525 may be composed of a suitably-resistant polymer such as acrylic, polyethersulfone, or polytetrafluoroethylene, and may be oleophobic and/or hydrophobic. In some embodiments, the filter 5525 may also comprise a supporting backing layer, for example a nonwoven polyester support. Preferably, the air leak 5524 will supply a relatively constant air flow that does not appreciably increase as additional negative pressure is applied to the system 5501. In embodiments of the suction adapter 5500 where the air flow through the air leak 5524 increases as additional negative pressure is applied, preferably this increased air flow will be minimized and not increase in proportion to the negative pressure applied thereto.

The filter 5525 provided in the controlled air leak 5524 in certain embodiments may be useful in a system 5501 for use with more ambulatory and active patients. For example, a chemically-resistant filter may permit a patient to bathe or shower without damaging the filter's functionality when reconnected to a source of negative pressure. Any occlusion or fluid blocking the air leak 5524 could then be cleared by, for example, wiping off the filter 5525 or re-applying negative pressure to the suction adapter 5500. Such a system would also have the advantage that the system 5501 and any assorted wound dressing materials, if present, would not need to be removed and then re-applied should a patient need to be disconnected from the source of negative pressure, for example incidental to bathing. This would entail significant advantages in improving the cost-effectiveness and ease of use of the present treatment system.

The suction adapter 5500 may be constructed so as to provide a consistent fluid flow even if the suction adapter 5500 is kinked or weighted down. For example, in use on a patient, the bridge portion 5502 may become folded over itself, or else the patient may roll over, thus placing his or her weight over at least a portion of the suction adapter 5500. Typically, prior art dressings and fluidic connectors become blocked or ineffective in such situations and in some cases may contribute to complications such as pressure ulcers. Here, however, certain embodiments provide for improved blockage resistance if kinked or weighed down. By employing channel layers 5512 and 5516 as described above, and by employing a foam channel layer 5512 and a fabric channel layer 5516, the suction adapter 5500 may be able to maintain a flow rate through the air leak 5524 of at least 0.08 L/min, and preferably 0.12 L/min while negative pressure is applied through a source of negative pressure. Further embodiments also provide for the suction adapter 5500 to be able to handle fluid exudate drainage from the wound site through the lower channel 5516 of at least 10 L/day, or 6.9 ml/min. Certain embodiments provide for the suction adapter 5500 to maintain these flow rates with a weight, for example a 12 kg weight, pressing down on the bridge portion through a rod with a 1 in. diameter. In some embodiments, these flow rates are also maintained while the bridge portion 5502 is kinked over itself with the same weight, or for example with a 4.75 kg weight placed directly on the folded region. It is preferable that the suction adapter 5500 be able to withstand being folded or kinked over even during an extended period of time, for example over 40 hours, and not show any degradation in performance (e.g., flow rates) compared to its performance prior to being folded or kinked over. Preferably, embodiments of the suction adapter 5500 are also able to transmit and maintain a negative pressure at the wound that is close to the negative pressure level at the source of negative pressure. For example, an acceptable level of pressure maintained at the wound may be within +-.25 mmHg of the negative pressure set at the source of negative pressure, with this pressure being preferably maintained at this level within 95% of the time that the suction adapter 5500 has negative pressure applied to it. Acceptable pressure levels may include pressure ranges between 40-120 mmHg, although levels of 200 mmHg have successfully been used.

With additional reference to Figs. 4A-5B and 6, the suction adapter 5500 may comprise an applicator 5520 designed for placement over a wound site. The applicator 5520 may comprise a flexible layer 5550, for example polyethylene or polyurethane, with a layer of adhesive on its lower (wound-facing) side. Optionally, a protective release layer 5529 may be placed on the adhesive layer, which is removable before use. In some embodiments, a more rigid removable backing layer 5552 may be provided on the upper side of the applicator 5520 to facilitate handling of the applicator 5520 due to the flexibility of the layer 5550. The applicator 5520 preferably comprises an attachment point for the bridge 5502 at the distal end 5505, for example using a section of double-sided adhesive tape 5528. The double-sided adhesive tape 5528 may be protected by an additional protective release layer, which is removed prior to adhering the bridge 5502 to the applicator 5520. It will be understood that different attachment methods are also contemplated, for example heat sealing, welding, or suitable adhesives. Some embodiments may also permit the manufacture of the bridge 5502 and the applicator 5520 as a single unit that does not require separate attachment means. The applicator 5520 preferably comprises at least one aperture 5526 through itself and designed to be placed over a wound site, and which can serve to fluidically connect the wound site to the source of negative pressure and to the air leak while also serving as a conduit to draw out wound exudate from the wound site.

In use, and with reference to Figs. 4A-B, the system 5501 may be used in a similar fashion to the other embodiments previously disclosed herein, such as the system 100 described in relation to Fig. 1. A wound site 5530 is preferably cleaned and prepared in a suitable fashion, and a wound packing material, if necessary, placed into the wound site, followed by a drape 5531. An aperture 5535 through the drape to the wound site is then created, although some embodiments may have a pre-made aperture 5535. Subsequently, an operator may situate the applicator portion 5520 over the aperture 5535. After removing the backing layer 5529 (if present) from the adhesive layer on the underside of the applicator portion 5520, the applicator is sealed to the drape 5531, and the backing layer 5552 (if present) is also removed from the applicator portion 5520. A fluidic conduit such as a tube 5540 may then be connected to the connector 5504. The tube 5540 may also be connected to connector 5504 prior to applying the applicator to the wound site. The fluidic conduit is connected to a source of negative pressure 5534, preferably with a container suitable for containing wound exudate interposed therebetween. The application of negative pressure may then be effectuated to the wound site 5530 until the wound site progresses to a desired level of healing.

During use of the system 5501, wound exudate from the wound site 5530 may be drawn by the negative pressure through the lower channel layer 5516. The air leak 5524 allows air to pass through the upper channel layer 5512 into the apertures through the distal ends of the layers 5512, 5514, 5516 and 5518. The negative pressure draws air passing through the upper channel layer into the lower channel layer 5516 back toward the source of negative pressure or pump. In some embodiments, the controlled air leak 5524 provides a constant flow of air through the suction adapter 5500, which then may be used to determine whether blockage or leakage is present. Causes of blockage can include, for example, situations where the lower channel 5516 becomes occluded with wound debris. Leakage causes can include, for example, improper sealing of the drape over the wound site, or physical damage to the suction adapter 5500 leading to excess air leaking into the system. The blockage or leakage may be determined, in certain embodiments, by measuring the speed of the pump while the pump works to maintain a constant negative pressure. Pump speed may also be measured indirectly by measuring the amount of voltage or signal sent to the pump.

### Multilayer Drape Optionally Configured for Alignment

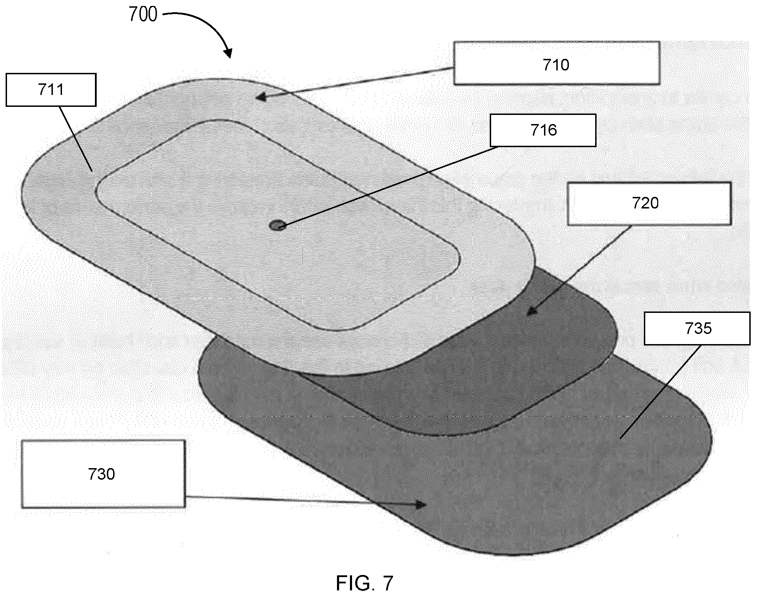

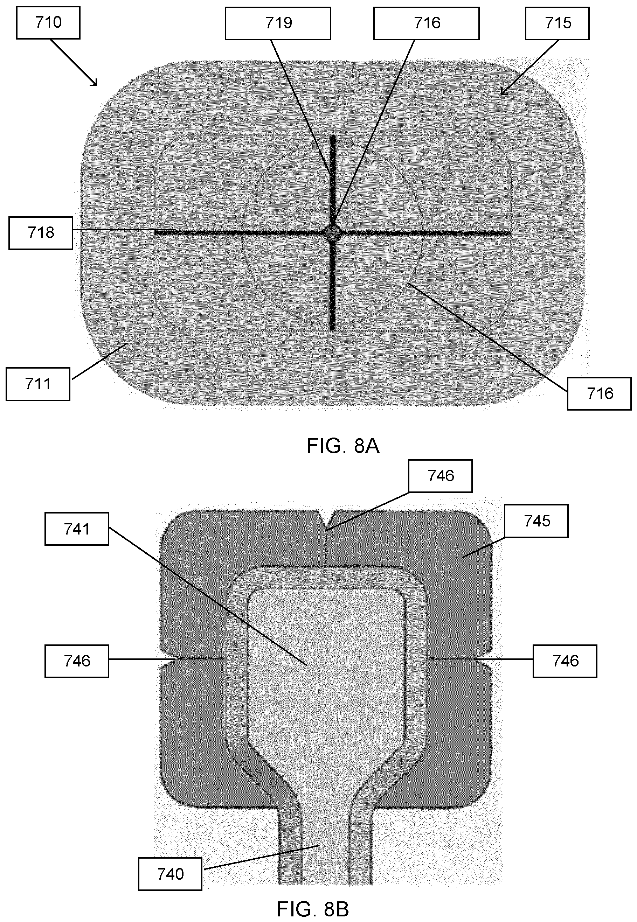

Figs. 7-9 illustrate configurations of a wound dressing or drape that can be used with any of the system configurations disclosed herein this section or elsewhere in the specification, such as in Figs 1-6. For example, such a drape could be incorporated as drape 5531 described above. Fig. 7 shows three layers of an embodiment of a drape design. The drape 700 includes a top layer 710 (also referred to as a "breathable top film"), an intermediate layer 720 (also referred to as a "spacer layer"), and a wound contact layer 730. The top layer 710 of the drape 700 can be formed of a breathable film that allows for fluid evaporation. The film can be transparent, such that from the top view of Figure 8A, other layers underneath the top layer 710 are also visible. The top layer 710 can include an adhesive for securing the dressing to the periwound area or the wound contact layer 730. The top layer 710 has a hole 716 configured to receive a port 740. In some embodiments, the hole 716 in the top layer 710 can be created during manufacturing. Alternatively, the hole 716 can be created by the user prior to positioning the drape 700 on the wound site and periwound site.

The intermediate layer 720, or the spacer layer 720, is one type of transmission layer that may be provided for communicating fluid through the drape 700. The intermediate layer 720 may have a smaller footprint than the top layer 710 and the wound contact layer 730, such that the intermediate layer 720 can be sandwiched between the top layer 710 and the wound contact layer 730. In some embodiments, the drape 700 can be manufactured with the top layer 710 and the wound contact layer 730 laminated together around the periphery via an adhesive layer (not shown). Alternatively, the top layer 710 may be directly laminated around the periphery (e.g., heat laminated) to the wound contact layer 730, without the need for an adhesive layer.

In some embodiments, the intermediate layer 720 can be a porous material. The intermediate layer 720 may allow transmission of fluid, including liquid and gas, away from a wound site into upper layers of the drape 700. In particular, the intermediate layer 720 can assist in distributing negative pressure evenly to the wound and periwound areas. This layer 720 may be formed of a material having a three-dimensional structure, for example, a knitted or woven spacer fabric (for example Baltex 7970 weft knitted polyester) or a non-woven fabric could be used. The three-dimensional material can comprise a 3D spacer fabric material similar to the material described in International Publication WO 2013/175306 A2 and International Publication WO2014/020440. The three-dimensional material may also be the same or similar to the materials described above for upper and lower channel layers 5512 and 5516 and may be comprised of a fabric, for example a knitted or woven spacer fabric (such as a knitted polyester 3D fabric, Baltex 7970.RTM., or Gehring 879.RTM.) or a nonwoven material. Suitable materials may also include terry-woven or loop-pile materials. The fibers may not necessarily be woven, and can include felted and flocked (including materials such as Flotex.RTM.) fibrous materials.

Some configurations of the intermediate layer 720 may additionally or alternatively comprise a wicking or acquisition distribution layer (ADL). The ADL is another type of transmission layer that may be provided for communicating fluid through the drape 700. The ADL may be configured to horizontally wick fluid such as wound exudate as it is absorbed upward through the layers of the drape 700. Lateral wicking of fluid may advantageously increase moisture vapor permeation and efficient delivery of negative pressure to the wound site. Some embodiments of the ADL may comprise viscose, polyester, polypropylene, polyethylene, cellulose (for example polysaccharide or repeated disaccharide), or a combination of some or all of these, and the material may be needle-punched. Some configurations of the ADL may comprise polyethylene in the range of 40-150 grams per square meter (gsm). Some configurations of the ADL may comprise a heavy fibrous melt material. Some configurations of the ADL may be relatively porous to allow for the passage of fluids, including gas, therethrough. One example of an ADL may comprise a lightweight, felt-like, viscose material, which may be 80 gsm (or approximately 80 gsm). Some embodiments of the ADL may comprise cellulose in the range of 40-160 gsm (or about 40 to about 160 gsm), for example 80 (or about 80) gsm. The ADL may be constructed from a material which resists compression under the levels of negative pressure commonly applied during negative pressure therapy. The ADL may be constructed so as to advantageously vertically wick fluid, such as wound exudate. Facilitating rapid movement of wound exudate from the transmission layer to the port 740 is desirable. Additionally judicious choice of material can reduce re- wetting of liquid from the upper layers down into lower layers, this phenomenon is known as "back wetting" or "re-wetting". Suitable materials that show an enhancement of this effect include Slimcore TL4 (150 gsm) from Libeltex BVBA or equivalent.

Some configurations of the ADL may include several internal layers. For example, one material suitable for use as an ADL includes a lower wicking or acquisition layer comprising substantially vertically extending fibers for vertical wicking of fluid and further includes an upper distribution layer comprising substantially horizontally extending fibers for horizontal wicking of fluid. Some ADL materials can include three or more layers, for example a lower wicking layer and two upper distribution layers. Other configurations can have one or more distribution layers positioned between upper and lower acquisition layers.

The wound contact layer 730 can comprise a flexible, biocompatible material, such as silicone. This layer 730 can further comprise a silicone adhesive on the skin-contact face and acrylic adhesive on the upper surface 735. In some embodiments, the wound contact layer 730 may be constructed from polyurethane, polyethylene or polyester. This layer 730 can further comprise a plurality of apertures, which can be created, for example, via a hot pin process, laser ablation process, ultrasound process or via another suitable method to be made permeable to liquid and gas. The apertures may comprise through holes in the wound contact layer 730 which enable fluid to flow through the layer 730. The array of apertures may comprise a shape selected from the group consisting of round, oval, triangular, square, rectangular, hexagonal, octagonal and any other polygonal shape. The wound contact layer 730 may prevent tissue ingrowth into the other material of the drape 700. The apertures may be are small enough to meet this requirement while still allowing fluid to flow there-through. For example, apertures formed as slits or holes having a size ranging from 0.025 mm to 1.2 mm are considered small enough to help prevent tissue ingrowth into the drape 700 while allowing wound exudate to flow into the drape 700. However, in certain embodiments, the apertures may be sized at least 0.5 mm, between 0.5 to 5 mm, or between 1 to 3 mm. The space between two adjacent holes can be in the range between 0.5 to 5 mm, between 0.5 to 3.5 mm, or between 1 to 3 mm. The thickness of the multi-care wound contact layer can be in the range of 1 to 10 mm, or 1 to 7 mm, or preferably 1.5 to 7 mm, or 1.5 to 4 mm, or 2 to 3 mm, or approximately 2 mm. In some configurations, the wound contact layer 730 may help maintain the integrity of the entire drape 700 while also creating an air tight seal around the intermediate layer 720 in order to maintain negative pressure at the wound.

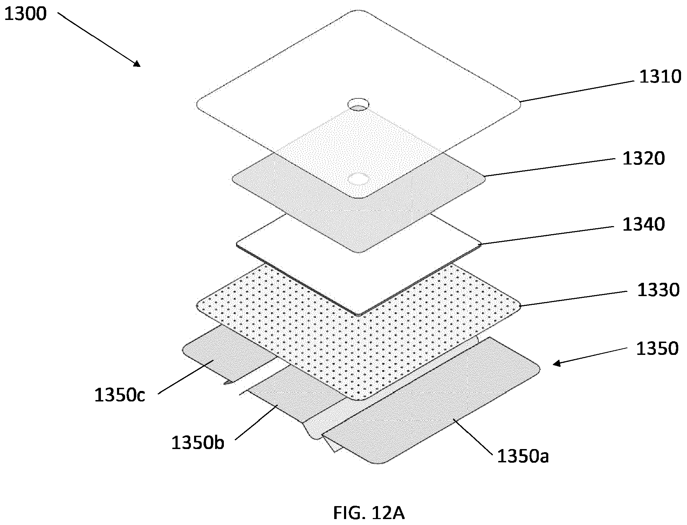

As shown in Fig. 7, all three layers 710, 720, 730 may overlap with a periwound area to allow for NPWT to that area. For example, the wound contact layer 730 can adhere to the periwound area surrounding a wound. The intermediate layer 720 can be positioned above the wound contact layer 730, which enables negative pressure to be distributed evenly across the periwound area. The top layer 710 can be positioned above the intermediate layer 720 and laminated to the wound contact layer 730, such that an air tight seal is created around the intermediate layer 720. When these overlapping layers 710, 720, 730 are placed on a periwound area, negative pressure can be applied to the top layer 710 and through the other two layers 720, 730 to the periwound area, which is described in further detail below. In some embodiments, additional layers such as described with respect to Figures 12A-16C may be provided between the top layer 710 and the wound contact layer 730. In some embodiments, the intermediate layer 720 is positioned in direct contact with the lower surface of the top layer 710. In some embodiments, one or more additional layers may be placed between the top layer 710 and the intermediate layer 720. In some embodiments, no absorbent layer is positioned between the top layer 710 and the wound contact layer 730, and/or between the top layer 710 and the intermediate layer 720.

In aspects according to the invention, referring to Figs. 8A-8B, the top layer 710 further comprises one or more placement guides 715 to aid the user in aligning a port 740 to the hole 716. As shown in Fig. 8A, the placement guides 715 can comprise two substantially perpendicular lines 718, 719 that intersect at the hole 716. Additionally, the placement guides 715 can include a circle 717 with the hole 716 at the center of the circle. It will be understood that other placement guides 715 are possible, including the use of multiple concentric circles with the hole 716 being at the center, the use of a square with the hole 716 at the center, and the use of dashed and solid lines. The placement guides 715 can be directly printed on the upper surface of the top layer 710. Alternatively, the placement guides 715 can be a sticker that is placed on the drape 700.

The drape 700 can be used with a port 740 that is configured to apply negative pressure to the drape 700, such as the Smith & Nephew RENASYS Soft Port or any port described herein this section or elsewhere in the specification, such as Figs. 4A-6. Referring to Fig. 8B, the port 740 may comprise one or more alignment features 745. These alignment features 745 are configured to interact with the placement guides 715 on the top layer 710. In one embodiment, the alignment features 745 comprise a generally square shape with a perimeter that extends beyond the head 741 of the port 740. The alignment features 745 can include one or more notches 746.

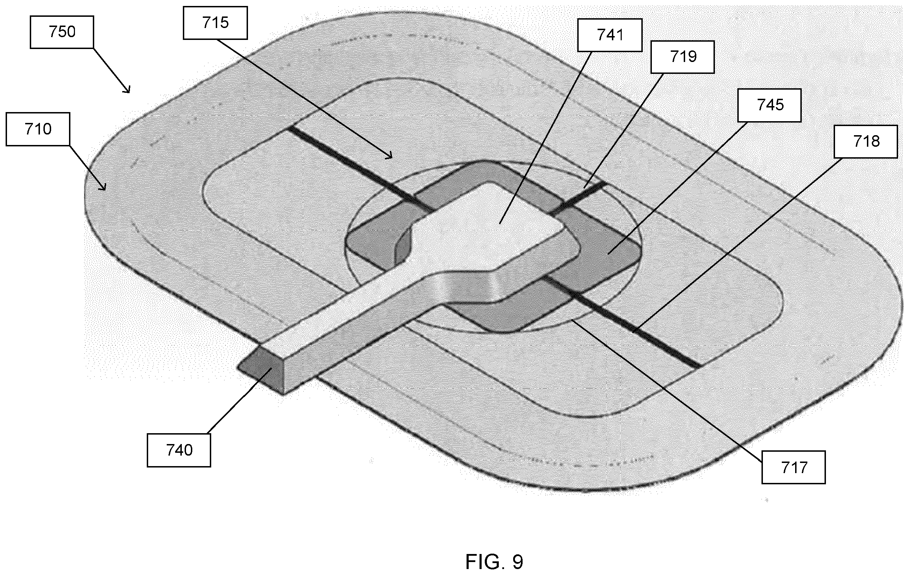

Fig. 9 shows the port 740 attached to the drape 700. When a user is positioning the port 740 onto the drape 700, the user can align the notches 746 with the substantially perpendicular lines 718, 719 on the top layer 710. This helps the user to align the hole 716 on the drape 700 with the hole (not shown) on the port 740. For situations where the port 740 may need to be rotated, the circle 717 on the drape 700 can be used to assist the user in aligning the two holes. As long as the corners of the alignment features 745 align with the marked circle 717 on the top layer 710 then the holes in both components are in line. It will be understood that other alignment features 745 maybe be used, for example, the alignment features 745 can comprise a circle with three notches 746 on the edges. In this embodiment, the user can align the notches 746 with the substantially perpendicular lines 718, 719 and align the edges of the circle with the circle 717 on the top layer 710.

### Method of Treating a Wound with a Multilayer Drape

Some embodiments described herein provide a method of treating a wound using wound dressings and drapes as described herein, such as the wound dressing or drape described above in relation to Figs 7-9. The method of treating a wound may comprise positioning a wound contact layer 730 in contact with the wound and the periwound area surrounding the wound. The wound contact layer 730 may comprise a flexible, biocompatible layer having a plurality of apertures extending through the wound contact layer 730. The flexible, biocompatible layer may comprise an elastomeric rubber, and a plurality of fluid-absorbent particles that are embedded in the flexible, biocompatible layer. The flexible, biocompatible layer may preferably further comprise a hydrophilic polymer. The fluid-absorbent particles can be configured to swell upon contact with fluid. Each of the fluid-absorbent particles may comprise a cross-linked polymer and an iodine-based antimicrobial agent, and may release the iodine-based antimicrobial agent upon the plurality of fluid-absorbent particles coming into contact with fluid from the wound.

A method of treating a wound may further comprise sizing the wound contact layer 730 to a size of the wound or periwound area before positioning the wound contact layer 730 in contact with the wound and the periwound area. Sizing the wound contact layer 730 may comprise cutting the wound contact layer 730 to match the size of the wound or periwound area. The wound contact layer 730 can be positioned in contact with the wound and periwound area with an adhesive adhered to the lower surface of the wound contact layer 730.

The method of treating a wound may further comprise, after positioning the wound contact layer 730 in contact with the wound and the periwound area, separately positioning an intermediate layer 720 over the wound contact layer 730 and a top layer 710 over the intermediate layer 720. Alternatively, the wound contact layer 730 can be integrated into a drape 700 comprising an intermediate layer 720 over the wound contact layer 730 and a top layer 710 over the intermediate layer 720. The wound contact layer 730 may have a perimeter shape that is substantially the same as or, alternatively, smaller than a perimeter shape of the top layer 710.

The method of treating a wound or locus as described above or described elsewhere herein may further comprise cutting a hole 716 in the center of the top layer 710. Alternatively, the top layer 710 can include a hole 716 at the center of the top layer 710. After the hole 716 is created, the user can align the alignment features 745 on the port 740 with the placement guides 715 on the top layer 710. For example, the user can align the notches 746 on the port 740 with the lines 718, 719 on the top layer 710. Additionally, the user can align the edges of the port 740 with the circle 717 on the top layer. After aligning the hole 718 on the top layer 710 with the hole on the head 741 of the port 740, the method further comprises delivering negative pressure through the top layer 710 to the wound and periwound area for a period of time.

In some alternative embodiments, the method of treating a wound may comprise using the drape 700 under ambient conditions not in connection with a negative pressure wound therapy system as described above, or described elsewhere herein.

### Multilayer Drape Optionally Configured with Handles

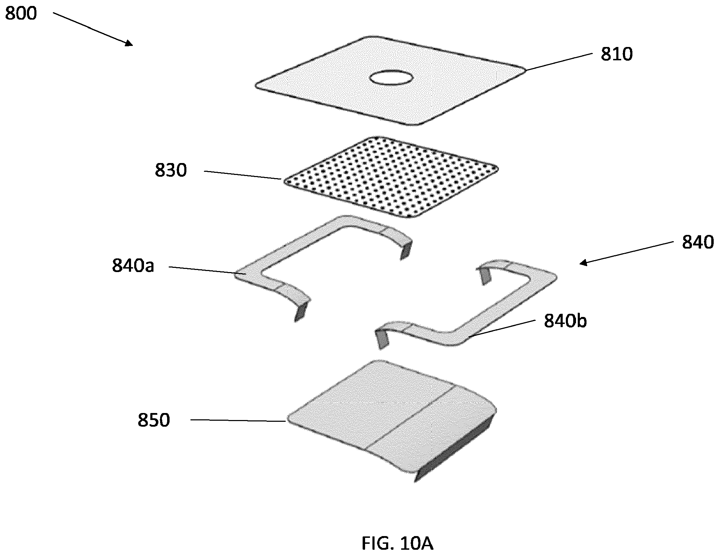

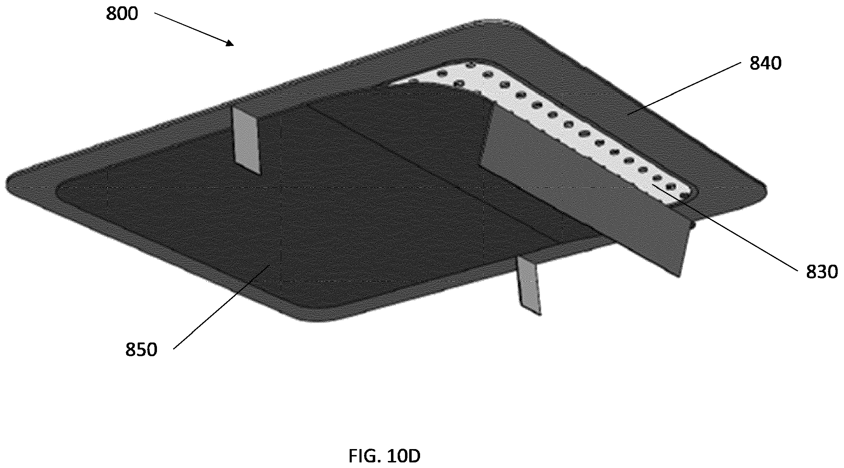

Figs. 10A-10D, 11A-11B, and 12A-12B illustrate different configurations of a wound dressing or drape that can be used with any of the system configurations disclosed herein this section or elsewhere in the specification, such as in Figs 1-6. For example, such a drape could be incorporated as drape 5531 described above.

With reference to Figs. 10A-10D, another configuration of a drape 800 is shown. The drape 800 resembles or is identical to the drape 700 discussed above in many respects. Any component or step disclosed in any configuration in this specification can be used in any other configuration.

Figs. 10A-10D shows a configuration of a drape design with two layers. The drape 800 can also include two protective layers. The drape 800 may include a top layer 810 and a wound contact layer 830. Although Figs. 10A-10D show the drape 800 with two layers, the drape 800 can include less than or more than two layers. The top layer 810 of the drape 800 can be formed of a breathable film that allows for fluid evaporation. The film can be transparent, such that from the top view of Fig. 10A, other layer(s) underneath the top layer 810 are also visible. The top layer 810 can include an adhesive for securing the dressing to the periwound area or the wound contact layer 830. For example, the top layer 810 can include an acrylic adhesive on a lower surface (i.e., the surface facing the wound contact layer 830) of the top layer 810.

The wound contact layer 830 may have a smaller footprint than the top layer 810, such that the top layer 810 extends beyond the wound contact layer 830 when the drape 800 is assembled. This configuration can reduce or eliminate the need for additional retention strips to secure the drape 800 to the patient. The wound contact layer 830 can comprise a flexible, biocompatible material, such as silicone. This layer 830 can further comprise a silicone adhesive on the skin-contacting surface and acrylic adhesive on the opposite surface. In some configurations, the wound contact layer 830 may be constructed from polyurethane, polyethylene or polyester. This layer 830 can further comprise a plurality of apertures, which can be created, for example, via a hot pin process, laser ablation process, ultrasound process or via another suitable method to be made permeable to liquid and gas. The apertures may comprise through holes in the wound contact layer 830 which enable fluid to flow through the layer 830. The array of apertures may comprise a shape selected from the group consisting of round, oval, triangular, square, rectangular, hexagonal, octagonal and any other polygonal shape. The wound contact layer 830 may prevent tissue ingrowth into the other material of the drape 800. The apertures may be small enough to prevent tissue ingrowth while still allowing fluid to flow there-through. For example, apertures formed as slits or holes having a size ranging from 0.025 mm to 1.2 mm may be small enough to impede or prevent tissue ingrowth into the drape 800 while allowing wound exudate to flow into the drape 800. However, in certain embodiments, the apertures may be sized at least 0.5 mm, between 0.5 to 5 mm, or between 1 to 3 mm. The space between two adjacent holes can be in the range between 0.5 to 5 mm, between 0.5 to 3.5 mm, or between 1 to 3 mm. The thickness of the multi-care wound contact layer can be in the range of 1 to 10 mm, or 1 to 7 mm, or preferably 1.5 to 7 mm, or 1.5 to 4 mm, or 2 to 3 mm, or approximately 2 mm. In some configurations, the wound contact layer 830 may help maintain the integrity of the entire drape 800 while also creating an air tight seal around the wound in order to maintain negative pressure at the wound.

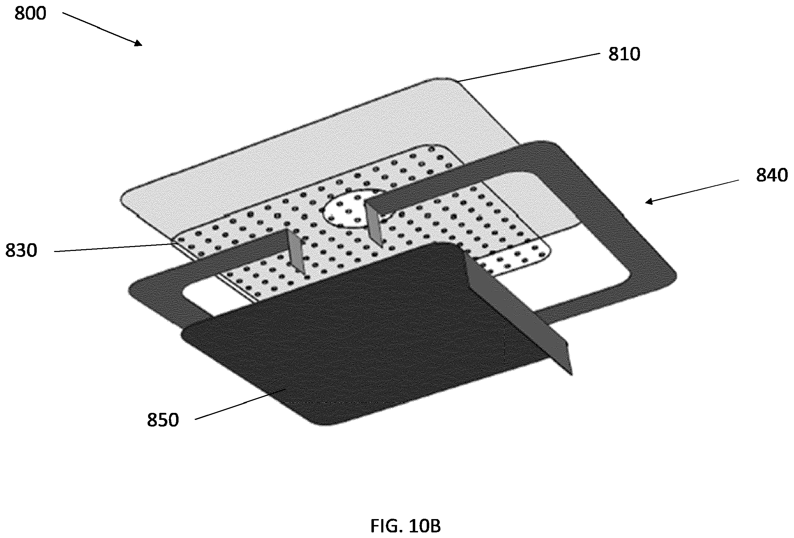

As shown in Figs. 10A and 10B, both layers 810, 830 may overlap with a periwound area to allow for delivery of NPWT to that area. For example, the wound contact layer 830 can adhere to the periwound area surrounding a wound. The top layer 810 can be positioned above the wound contact layer 830 such that the portions of the top layer 810 that extends beyond the wound contact layer 830 can adhere to the periowound area. When these overlapping layers 810, 830 are placed on a periwound area, negative pressure can be applied to the top layer 810 and through the wound contact layer 830 to the periwound area, which is described in further detail below.

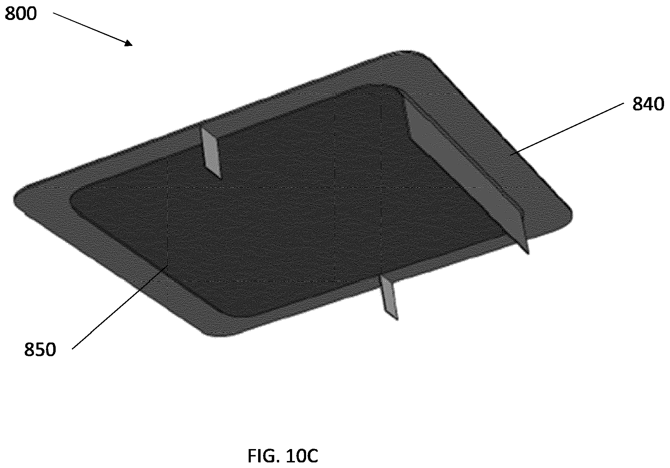

The drape 800 may include two protective release layers: a first protective release layer 840 comprising a pair of protective release strips 840a, 840b and a second protective release layer 850. The protective release layers 840, 850 may be removable before use. The pair of protective release strips 840a, 840b may be removably attached to the lower surface of the top layer 810 to maintain the adhesiveness of the acrylic adhesive of the top layer 810. The second protective release layer 850 may be removably attached to the skin-contacting surface of the wound contact layer 830 to maintain the adhesiveness of the silicone adhesive of the wound contact layer 830. As shown in Figs. 10C-10D, the second protective release layer 850 can be positioned in the center of the dressing 800. The second protective release layer 850 may be positioned below and may overlap the first protective release layer 840, such that the second protective release layer 850 is configured to be removed first in use. One of skill in the art will understand that the protective release strips may be broken up into further portions such as one, three, four, five or more strips and the one or more strips may be positioned around the entire periphery of the drape or a lesser portion thereof.

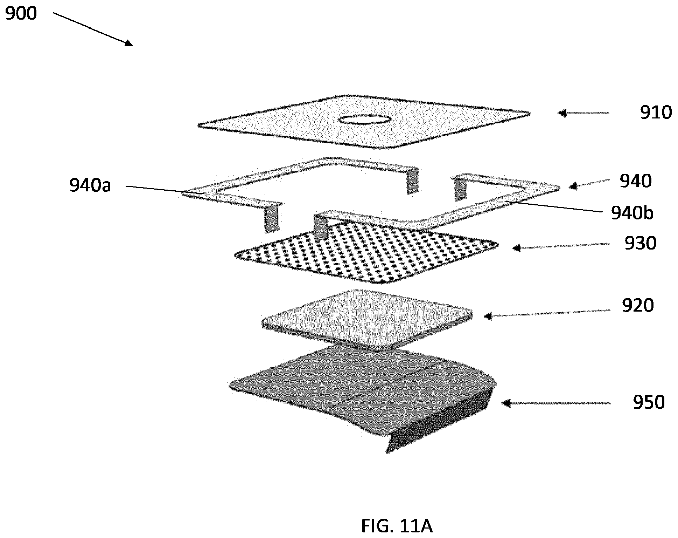

With reference to Figs. 11A-11B, another configuration of a drape 900 is shown. The drape 900 resembles or is identical to the drape 700 discussed above in many respects. Any component or step disclosed in any configuration in this specification can be used in any other configuration.

Figs. 11A-11B shows a configuration of a drape design with three layers. The drape 900 can also include two protective layers. The drape 900 may include a top layer 910, a spacer layer 920, and a wound contact layer 930. Although Figs. 11A-11B show the drape 900 with three layers, the drape 900 can include less than or more than three layers. The top layer 910 of the drape 900 can be formed of a breathable film that allows for fluid evaporation. The film can be transparent, such that from the top view of Fig. 11A, other layer(s) underneath the top layer 910 are also visible. The top layer 910 can include an adhesive for securing the dressing to the periwound area or the wound contact layer 930. For example, the top layer 910 can include an acrylic adhesive on a lower surface (i.e., the surface facing the wound contact layer 930) of the top layer 910.

The wound contact layer 930 may have a smaller footprint than the top layer 910, such that the top layer 910 extends beyond the wound contact layer 930 when the drape 900 is assembled. This configuration can reduce or eliminate the need for additional retention strips to secure the drape 900 to the patient. The wound contact layer 930 can comprise a flexible, biocompatible material, such as silicone. This layer 930 can further comprise a silicone adhesive on the skin-contacting surface and acrylic adhesive on the opposite surface. In some configurations, the wound contact layer 930 may be constructed from polyurethane, polyethylene or polyester. This layer 930 can further comprise a plurality of apertures, which can be created, for example, via a hot pin process, laser ablation process, ultrasound process or via another suitable method to be made permeable to liquid and gas. The apertures may comprise through holes in the wound contact layer 930, which enable fluid to flow through the layer 930. The array of apertures may comprise a shape selected from the group consisting of round, oval, triangular, square, rectangular, hexagonal, octagonal and any other polygonal shape. The wound contact layer 930 may prevent tissue ingrowth into the other material of the drape 900. The apertures may be small enough to prevent tissue ingrowth while still allowing fluid to flow there-through. For example, apertures formed as slits or holes having a size ranging from 0.025 mm to 1.2 mm may be small enough to impede or prevent tissue ingrowth into the drape 900 while allowing wound exudate to flow into the drape 900. However, in certain embodiments, the apertures may be sized at least 0.5 mm, between 0.5 to 5 mm, or between 1 to 3 mm. The space between two adjacent holes can be in the range between 0.5 to 5 mm, between 0.5 to 3.5 mm, or between 1 to 3 mm. The thickness of the multi-care wound contact layer can be in the range of 1 to 10 mm, or 1 to 7 mm, or preferably 1.5 to 7 mm, or 1.5 to 4 mm, or 2 to 3 mm, or approximately 2 mm. In some configurations, the wound contact layer 930 may help maintain the integrity of the entire drape 900 while also creating an air tight seal around the wound in order to maintain negative pressure at the wound.

The spacer layer 920 is one type of transmission layer that may be provided for communicating fluid through the drape 900. The spacer layer 920 may have a smaller footprint than the top layer 910 and the wound contact layer 930, such that the top layer 910 and the wound contact layer 930 extend beyond the spacer layer 920 when the drape 900 is assembled. In some configurations, the spacer layer 920 can be a porous material. The spacer layer 920 may allow transmission of fluid, including liquid and gas, away from a wound site into upper layers of the drape 900. In particular, the spacer layer 920 can assist in distributing negative pressure evenly to the wound and periwound areas. This layer 920 may be formed of a material having a three-dimensional structure, for example, a knitted or woven spacer fabric (for example Baltex 7970 weft knitted polyester) or a non-woven fabric could be used.

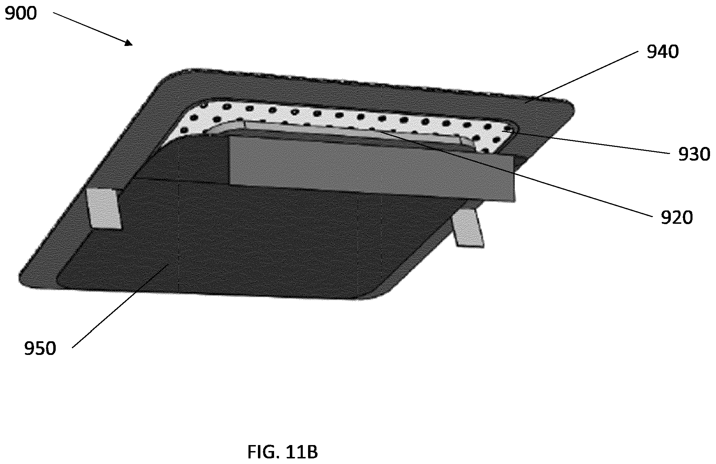

As shown in Figs. 11A and 11B, the layers 910, 920, 930 may overlap with a periwound area to allow for NPWT to that area. For example, the spacer layer 920 may be positioned on a wound site and the wound contact layer 930 can be positioned over the spacer layer 920. The portions of the wound contact layer 930 that extend beyond the spacer layer 920 can adhere to the periwound area surrounding the wound site. The top layer 910 can be positioned above the wound contact layer 930 such that the portions of the top layer 910 that extend beyond the wound contact layer 930 can adhere to the periwound area. When these overlapping layers 910, 920, 930 are placed on a periwound area, negative pressure can be applied to the top layer 910 and through the wound contact layer 930 and the spacer layer 920 to the periwound area, which is described in further detail below.

The drape 900 may include two protective release layers: a first protective release layer 940 comprising a pair of protective release strips 940a, 940b and a second protective release layer 950. The protective release layers 940, 950 may be removable before use. The pair of protective release strips 940a, 940b may be removably attached to the lower surface of the top layer 910 to maintain the adhesiveness of the acrylic adhesive of the top layer 910. The second protective release layer 950 can be positioned underneath the spacer layer 920 and the wound contact layer 930. The second protective release layer 950 may be removably attached to the portions of the wound contact layer 930 that extend beyond the spacer layer 920 to maintain the adhesiveness of the silicone adhesive of the wound contact layer 930. As shown in Fig. 11B, the second protective release layer 950 can be positioned in the center of the dressing 900. The second protective release layer 950 may be positioned below and may overlap the first protective release layer 940, such that the second protective release layer 950 is configured to be removed first in use.

With reference to Figs. 12A-12B, another configuration of a wound dressing or drape 1300 is shown. The drape 1300 resembles or is identical to the drape 700, 900 discussed above in many respects. Any component or step disclosed in any configuration in this specification can be used in any other configuration.

Figs. 12A-12B shows a configuration of a drape design with four layers. The drape 1300 can also include at least one protective layer 1350. The drape 1300 may include a top layer 1310, an intermediate layer 1320 (also referred to herein as a protective construction layer or PCL), a transmission layer 1340, and a wound contact layer 1330. The top layer 1310, the intermediate layer 1320, wound contact layer 1330, and the transmission layer 1340 can comprise any of a number of shapes, such as a square, a rectangular, or an oval. Moreover, the top layer 1310 can be positioned over the intermediate layer 1320 with the transmission layer 1340 being positioned between the intermediate layer 1320 and the wound contact layer 1330. Although Figs. 12A-12B show the drape 1300 with four layers and a certain positioning of the layers, the drape 1300 can include less than or more than four layers and the layers can be arranged in a different order.

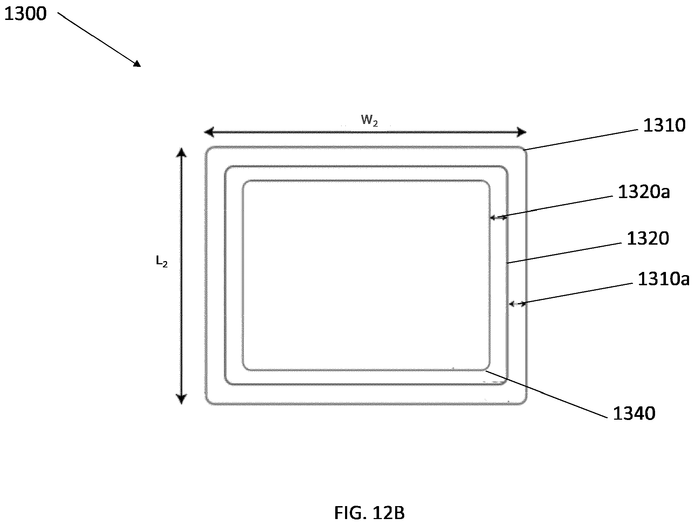

The top layer 1310 of the drape 1300 can be formed of a breathable film that allows for fluid evaporation, for example polyurethane. The film can be transparent, such that from the top view of Fig. 12A (e.g., Fig. 12B), other layer(s) underneath the top layer 1310 are also visible. The top layer 1310 can include an adhesive for securing the top layer 1310 to the intermediate layer 1320 and the wound contact layer 1330. Additionally, or alternatively, the adhesive of the top layer 1310 can secure the dressing 1300 to the periwound area, as described above. For example, the top layer 1310 can include an acrylic adhesive on a lower surface (i.e., the surface facing the other layers 1320, 1330, 1340) of the top layer 1310.

As shown in Fig. 12B, the top layer 1310 can include a width W₂ and a length L₂. The width W₂ can be approximately 220 mm, approximately 270 mm, or approximately 420 mm. The width W₂ can be between approximately 150 mm to approximately 500 mm, approximately 200 mm to approximately 450 mm, approximately 250 mm to approximately 400 mm, approximately 300 mm to approximately 350 mm, or any suitable width. The length L₂ can be approximately 170 mm or approximately 270 mm. The length L₂ can be between approximately 100 mm to approximately 300 mm, approximately 125 mm to approximately 275 mm, approximately 150 mm to approximately 250 mm, approximately 175 mm to approximately 225 mm, or any suitable length.

In some configurations, the top layer 1310 can include a greater width and a greater length than the other layers 1320, 1330, 1340 such that the top layer 1310 includes a top border 1310a that extends beyond the perimeter of the other layers 1320, 1330, 1340. In this configuration, the top border 1310a can adhere to the periwound area in use. In some configurations, the top layer 1310 and the wound contact layer 1330 can have the same length and width such that the top layer 1310 adheres to the wound contact layer 1330 to sandwich the other layer 1320, 1340 between the two layers 1310, 1330. In this configuration, the wound contact layer 1330 can adhere to the wound site and the periwound area in use. The top border 1310a can include a width that can be measured from an edge of the PCL 1320 to an edge of the top layer 1310. The width can be approximately 30 mm. The width can be between approximately 10 mm to approximately 50 mm, approximately 15 mm to approximately 45 mm, approximately 20 mm to approximately 40 mm, approximately 25 mm to approximately 35 mm, or any suitable width.

The size of the top layer 1310 can correspond with a size of the dressing, such as small, medium or large. The table below shows example dimensions of the top layer 1310 that can correspond with the small, medium or large sizes.

| **Size** | **Top Border 1310a width** | **Length L₂** | **Width W₂** |
|---|---|---|---|
| Small | 30 mm | 170 mm | 220 mm |
| Medium | 30 mm | 270 mm | 270 mm |
| Large | 30 mm | 270 mm | 420 mm |

The intermediate layer 1320 can be a protective construction layer (PCL) 1320 configured to provide support to the dressing 1300 and protect the top layer from the transmission layer 1340 (e.g., from being pierced by monofilaments of the transmission layer 1340 when the transmission layer comprises monofilaments). For example, the PCL 1320 can provide a layer of support between the top layer 1310 and the transmission layer 1340. The PCL 1320 can be configured to act as a masking or obscuring layer to help reduce the unsightly appearance of a drape 1300 during use due to the absorption of wound exudate. The PCL 1320 may be a colored portion of the transmission layer 1340, or may be a separate layer that covers the transmission layer 1320. The PCL 1320 may be one of a variety of colors such as blue, orange, yellow, green, or any color suitable for masking the presence of wound exudate in the drape 1400. For example, a blue PCL 1320 may be a shade of blue similar to the shade of blue commonly used for the material of medical gowns, scrubs, and drapes. Some configurations of the PCL 1320 may comprise polypropylene spunbond material. Further, some configurations of the PCL 1320 may comprise a hydrophobic additive or coating. Some configurations may comprise a thin fibrous sheet of 60, 70, or 80 gsm.

The PCL 1320 may comprise at least one viewing window configured to allow a visual determination of the saturation level of the transmission layer 1340. The at least one viewing window may comprise at least one aperture made through the PCL 1320. The at least one viewing window may comprise at least one uncolored region of the PCL 1320. Some configurations of the PCL 1320 may comprise a plurality of viewing windows or an array of viewing windows.

The masking capabilities of the PCL 1320 can be partial or less than 100% obscuring to thereby allow clinicians to access the information they require by observing the spread of exudate across the dressing surface. The masking capabilities of the PCL 1320 may be partial due to material properties allowing wound exudate to slightly alter the appearance of the dressing 1300 or due to the presence of at least one viewing window in a completely obscuring material. The partial masking nature of the PCL 1320 may enable a skilled clinician to perceive a different colour caused by exudate, blood, by-products etc. in the dressing allowing for a visual assessment and monitoring of the extent of spread across the dressing. However, since the change in colour of the dressing from its clean state to a state with exudate contained may only be a slight change, the patient is unlikely to notice any aesthetic difference. Reducing or eliminating a visual indicator of wound exudate from a patient is likely to have a positive effect on their health, reducing stress for example.

The PCL 1320 can include a width and a length. As shown in Fig. 12B, the length and width of the PCL 1320 can be less than the width W₂ and length L₂ of the top layer 1310, as described above. The width of the PCL 1320 can be approximately 160 mm, approximately 210 mm, or approximately 360 mm. The width can be between approximately 100 mm to approximately 400 mm, approximately 125 mm to approximately 375 mm, approximately 150 mm to approximately 350 mm, approximately 200 mm to approximately 325 mm, approximately 225 mm to approximately 300 mm, approximately 250 mm to approximately 300 mm, or any suitable width. The length of the PCL 1320 can be approximately 110 mm or approximately 210 mm. The length can be between approximately 50 mm to approximately 300 mm, approximately 75 mm to approximately 275 mm, approximately 100 mm to approximately 250 mm, approximately 125 mm to approximately 225 mm, approximately 150 mm to approximately 200 mm, or any suitable length.

In some configurations, the PCL 1320 can include a greater width and a greater length than the transmission layer 1340 such that the PCL 1320 includes a PCL border 1320a that extends beyond the perimeter of the transmission layer 1340. The PCL border 1320a can include a width that can be measured from an edge of the transmission layer 1340 to an edge of the PCL 1320. The width can be approximately 5 mm. The width can be between approximately 5 mm to approximately 20 mm, or any suitable width.

The size of the PCL 1320 can correspond with a size of the dressing, such as small, medium or large. The table below shows example dimensions of the PCL 1320 that can correspond with the small, medium or large sizes.

| **Size** | **PCL border 1320a width** | **PCL 1320 Length** | **PCL 1320 Width** |
|---|---|---|---|
| Small | 5 mm | 110 mm | 160 mm |
| Medium | 5 mm | 210 mm | 210 mm |
| Large | 5 mm | 210 mm | 360 mm |

The transmission layer 1340 can be configured to act as a transmission layer similar to the intermediate layer 720 describe above in relation to Figs. 7-9. Additionally, the transmission layer 1340 can include a 3D fabric material or any of the materials as described above for the intermediate layer 720. In some embodiments, the transmission layer may additionally or alternatively comprise an absorbent material, and in other embodiments, the transmission layer may not have any absorbent material. If absorbent material is utilized, the absorbent material may be a foam or non-woven natural or synthetic material and which may optionally include or be super-absorbent material forms a reservoir for fluid, particularly liquid, removed from the wound site. The material of the transmission layer 1340 also prevents liquid collected in the drape 1300 from flowing in a sloshing manner. The transmission layer 1340 can help distribute fluid throughout the layer 1340 via a wicking action so that fluid is drawn from the wound site and stored throughout the transmission layer 1340. This can help prevent agglomeration in areas of the transmission layer 1340. The capacity of the absorbent material must be sufficient to manage the exudates flow rate of a wound when negative pressure is applied. Since in use the transmission layer 1340 experiences negative pressures the material of the transmission layer 1340 is chosen to absorb liquid under such circumstances. A number of materials exist that are able to absorb liquid when under negative pressure, for example superabsorber material. In addition to materials described above, the transmission layer 1340 may be manufactured from or comprise ALLEVYN^{™} foam, Freudenberg 114-224-4 and/or Chem-Posite^{™} 11C-450.

In some configurations, the transmission layer 1340 can include non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers may lead to strong capillary action in the fibrous pad which helps distribute liquid. In this way, the super-absorbent material is efficiently supplied with liquid. Also, all regions of the transmission layer 1340 are provided with liquid. The wicking action also assists in bringing liquid into contact with the top layer 1310 to aid increase transpiration rates of the drape 1300. The wicking action may also assist in delivering liquid downwards towards the wound bed when exudation slows or halts. This delivery process can help maintain the transmission layer 1340 and lower wound bed region in a moist state which helps prevent crusting within the drape 1300, which could lead to blockages, and helps maintain an environment optimized for wound healing.

In some configurations, the transmission layer 1340 may include an air-laid material or any suitable material disclosed herein. Heat fusible fibers may optionally be used to assist in holding the structure of the drape 1300 together. It will be appreciated that rather than using super-absorbing particles or in addition to such use, super-absorbing fibers may be utilized according to certain configurations of the present disclosure. An example of a suitable material is the Product Chem-Posite^{™} 11 C available from Emerging Technologies Inc (ETi) in the USA.

Optionally, according to certain configurations of the present disclosure, the transmission layer 1340 may include synthetic stable fibers and/or bi-component stable fibers and/or natural stable fibers and/or super-absorbent fibers. Fibers in the transmission layer 1040 may be secured together by latex bonding or thermal bonding or hydrogen bonding or a combination of any bonding technique or other securing mechanism. In some configurations, the transmission layer 1340 may be formed by fibers which operate to lock super-absorbent particles within the transmission layer 1340. Such an arrangement can help ensure that super-absorbent particles do not move external to the transmission layer 1340 and towards an underlying wound bed. This may improve functionality because when negative pressure is applied there is a tendency for the transmission layer 1340 to collapse downwards and this action may push super-absorbent particle matter into a direction towards the wound bed if they were not locked away by the fibrous structure of the transmission layer 1340.

The transmission layer 1340 may comprise multiple fibers. The fibers can be strand-like and made from cellulose, polyester, viscose or the like. Dry absorbent particles can be distributed throughout the transmission layer 1340 ready for use. In some configurations, the transmission layer 1340 includes a pad of cellulose fibers and a plurality of super absorbent particles. In additional configurations, the transmission layer 1040 can be a non-woven layer of randomly orientated cellulose fibers.

The transmission layer 1340 may include a length and a width. The width of the transmission layer 1340 can be approximately 150 mm, approximately 200 mm, or approximately 350 mm. The width can be between approximately 100 mm to approximately 400 mm, approximately 150 mm to approximately 350 mm, approximately 200 mm to approximately 300 mm, or any suitable width. The length of the transmission layer 1340 can be approximately 100 mm or approximately 200 mm. The length can be between approximately 50 mm to approximately 300 mm, approximately 75 mm to approximately 275 mm, approximately 100 mm to approximately 250 mm, approximately 125 mm to approximately 225 mm, approximately 150 mm to approximately 200 mm, or any suitable length.

The size of the transmission layer 1340 can correspond with a size of the dressing, such as small, medium or large. The table below shows example dimensions of the transmission layer 1340 that can correspond with the small, medium or large sizes.

| **Size** | **Transmission layer 1340 Length** | **Transmission layer 1340 Width** |
|---|---|---|
| Small | 110 mm | 160 mm |
| Medium | 210 mm | 210 mm |
| Large | 210 mm | 360 mm |

The wound contact layer 1330 may be the same as or similar to the wound contact layers 830, 930 described above in relation to Figs. 10A-11B. The wound contact layer 1330 may include a length and a width. The width of the wound contact layer 1330 can be approximately 220 mm, approximately 270 mm, or approximately 420 mm. The width can be between approximately 200 mm to approximately 500 mm, approximately 250 mm to approximately 450 mm, approximately 300 mm to approximately 400 mm, or any suitable width. The length of the wound contact layer 1330 can be approximately 170 mm or approximately 270 mm. The length can be between approximately 100 mm to approximately 300 mm, approximately 125 mm to approximately 275 mm, approximately 150 mm to approximately 250 mm, approximately 175 mm to approximately 225 mm, or any suitable length.

In some configurations, the wound contact layer 1330 can be the same size as the top layer 1310. In this configuration, the wound contact layer 1330 can adhere to the wound site and the periwound area in use. The wound contact layer 1330 may include a wound contact border (not shown). The wound contact border may be the same size or a different size as the top border 1310a. The wound contact border can include a width that can be measured from an edge of the PCL 1320 to an edge of the wound contact layer 1330. The width can be approximately 30 mm. The width can be between approximately 10 mm to approximately 50 mm, approximately 15 mm to approximately 45 mm, approximately 20 mm to approximately 40 mm, approximately 25 mm to approximately 35 mm, or any suitable width.

The size of the wound contact layer 1330 can correspond with a size of the dressing, such as small, medium or large. The table below shows example dimensions of the wound contact layer 1330 that can correspond with the small, medium or large sizes.

| **Size** | **Wound Contact Border width** | **Wound Contact Layer 1330 Length** | **Wound Contact Layer 1330 Width** |
|---|---|---|---|
| Small | 30 mm | 170 mm | 220 mm |
| Medium | 30 mm | 270 mm | 270 mm |
| Large | 30 mm | 270 mm | 420 mm |

As shown in Figs. 12A and 12B, the layers 1310, 1320, 1330, 1340 may overlap with a periwound area to allow for NPWT to that area. For example, the wound contact layer 1330 may be positioned on a wound site and the transmission layer 1340 can be positioned over the wound contact layer 1330. The wound contact layer 1330 can adhere to the periwound area surrounding the wound site. The PCL 1320 can be positioned over the transmission layer 1340 such that the PCL 1320 can reduce the risk of the material from the transmission layer 1340 piercing the top layer 1310. The top layer 1310 can be positioned above the PCL 1320 such that the portions of the top layer 1310 that extend beyond the PCL 1320 and the transmission layer 1340 can adhere to the wound contact layer 1330 and/or the periwound area. When these overlapping layers 1310, 1320, 1330, 1340 are placed on a periwound area, negative pressure can be applied to the top layer 1010 and through the other layer 1320, 1330, 1340 to the periwound area, which is described in further detail below. An opening or aperture may be provided in the top layer 1310 and in the PCL 1320, such as in a center of these layers, to provide negative pressure to the wound site and to the periwound area through the drape, such as through a port or suction adapter as described in this specification.

The drape 1300 may include at least one protective release layer 1350 that can be the same as or similar to the first and second protective release layers 840, 850, 940, 950 discussed above in relation to Figs. 10A-11B. The at least one protective release layer 1350 may comprise a single integral component or several overlapping components. The illustrated configuration in Fig. 12A has three overlapping components: a center release strip 1350b and first and second side release strips 1350a, 1350c. Although three release strips are shown, the drape 1300 can include more or less than three release strips. The at least one protective release layer 1350 may be removable before use. For example, each of the release strips 1350a, 1350b, 1350c may include a handle and/or a pair of handles, which can be configured to remove the strips when actuated 1350a, 1350b, 1350c. A user, for example, can pull on the handle(s) to remove the strips 1350a, 1350b, 1350c. The at least one protective release layer 1350 may be removably attached to the lower surface of the wound contact layer 1330 and/or the top layer 1310. As shown in Fig. 12A, the first and second release strips 1350a, 1350c can be positioned on opposite sides of the center release strips 1350b. The first and second release strips 1350a, 1350c can overlap opposite sides of the center release strips 1350b

### Method of Treating a Wound with a Multilayer Drape

Some embodiments described herein provide a method of treating a wound using wound dressings and drapes as described herein, such as the wound dressing or drape 800, 900, 1300 described above in relation to Figs 10A-12B. The method of treating a wound may comprise removing the second protective layer 850, 950 or the center release strip 1350b from the drape 800, 900, 1300. The drape 800, 900, 1300 can be positioned on the wound such that the wound contact layer 830, 930, 1330 and/or the spacer layer 920 contacts the wound and/or a periwound area surrounding the wound. The wound contact layer 830, 930, 1330 can be adhered to the wound and/or the periowound area. The drape 800, 900, 1300 can optionally be repositioned over the wound and periwound area until the drape 800, 900, 1300 is in a proper position to provide effective treatment to the wound. The pair of protective release strips 840a, 840b, 940a, 940b or the first and second side release strips 1350a, 1350c can be removed from the adhesive surface of the top layer 810, 910, 1310. The portions of the top layer 810, 910, 1310 that extend over the wound contact layer 830, 930, 1330 can be adhered to the periwound area such that the drape 800, 900, 1300 is secured to the patient such that negative pressure can be applied to the wound and periwound area through the drape 800, 900, 1300.

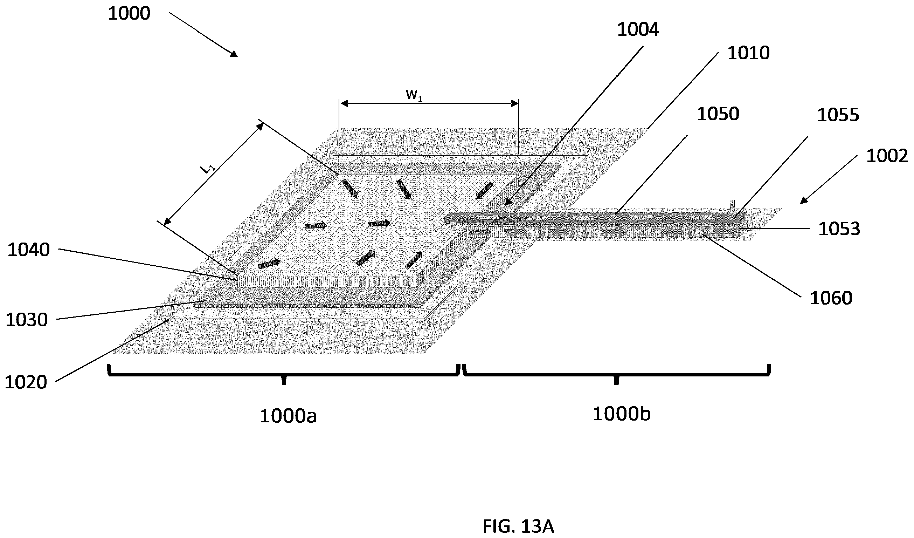

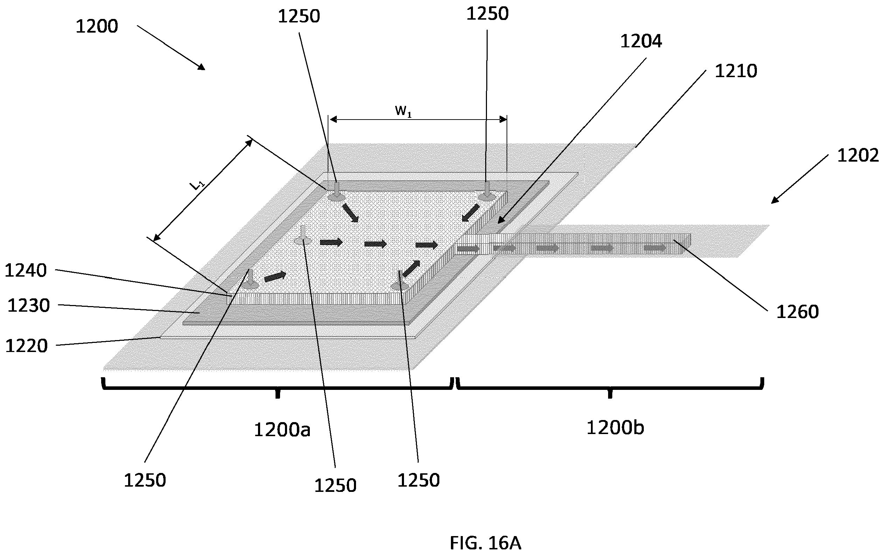

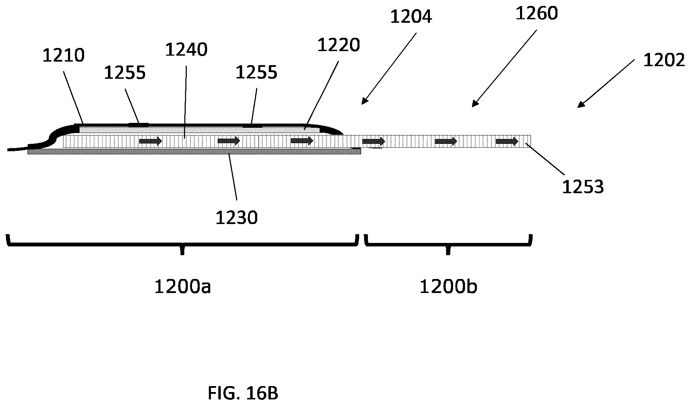

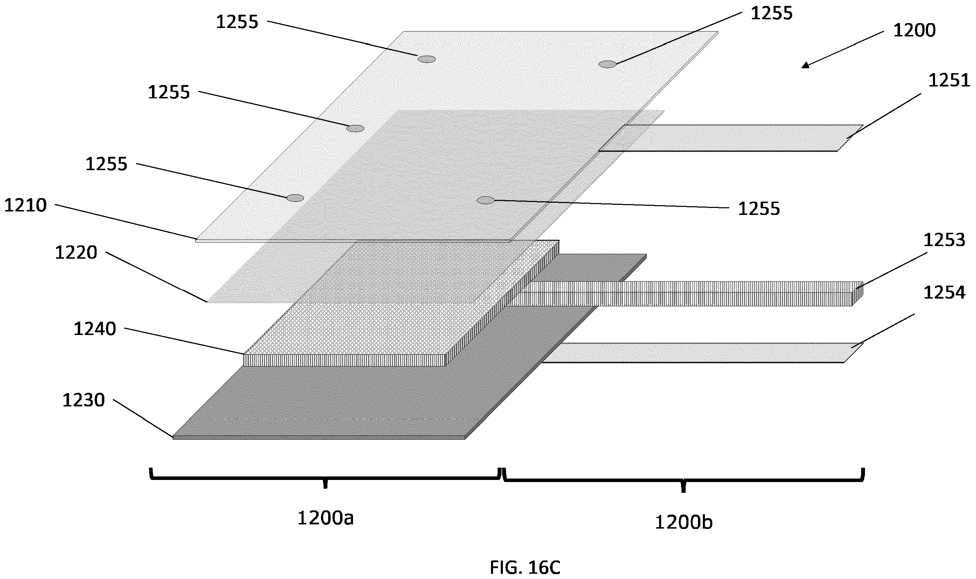

Some embodiments described herein provide a method of treating a wound using wound dressings and drapes as described herein, such as the drape 1300 described above in relation to Figs 12A-12B. The method of treating a wound may comprise removing the at least one protective release layer 1350. The drape 1300 can be positioned on the wound such that the wound contact layer 1330 contacts the wound and/or a periwound area surrounding the wound. The wound contact layer 1330 can be adhered to the wound and/or the periowound area. The drape 1300 can optionally be repositioned over the wound and periwound area until the drape 1300 is in a proper position to provide effective treatment to the wound. A port or suction adapter as described elsewhere in this specification may be utilized to supply negative pressure to the drape 1300. The port or suction adapter may be pre-applied to the drape over the opening in the top layer 1310 before the drape is positioned on the wound, or it may be applied after the drape is positioned on the wound. In other embodiments such as described with respect to Figs. 13A-16C, a port or suction adapter (described as a drape tail) may be integrally formed with the drape 1300 described above.

In some alternative configurations, the methods of treating a wound may comprise using the drape 800, 900, 1300 under ambient conditions not in connection with a negative pressure wound therapy system as described above, or described elsewhere herein.

### Multilayer Drape Optionally for use with an Integrated Port

Figs. 13A-16C illustrate different configurations of a dressing that can be used with any of the system configurations disclosed herein this section or elsewhere in the specification, such as in Figs 1-6. For example, such a dressing could be incorporated as drape 5531 described above. Some of the views shown in Figs. 13A-16C may not show all of the layers in the different configurations of the dressing for ease of illustration.

With reference to Figs. 13A-14, a drape 1000 is shown. The drape 1000 resembles or is identical to the drape 700 discussed above in many respects. Any component or step disclosed in any configuration in this specification can be used in any other configuration.

Figs. 13A-14 shows a drape 1000 including a drape portion 1000a with four layers and a drape tail 1000b. The drape portion 1000a may include a top layer 1010, an intermediate layer 1020, a wound contact layer 1030, and a transmission layer 1040. The top layer 1010, the intermediate layer 1020, wound contact layer 1030, and the transmission layer 1040 can comprise any of a number of shapes, such as a square, a rectangular, or an oval.

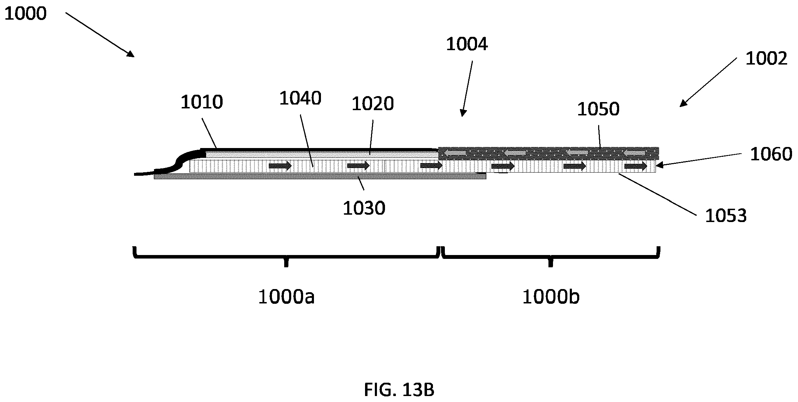

As shown in Figs. 13B-13C, the top layer 1010 can be positioned over the intermediate layer 1020 with the transmission layer 1040 being positioned between the intermediate layer 1020 and the wound contact layer 1030. Although Figs. 13A-14 show the drape 1000 with four layers and a certain positioning of the layers, the drape 1000 can include less than or more than four layers and the layers can be arranged in a different order. The top layer 1010 of the drape 1000 can be formed of a breathable film that allows for fluid evaporation. The top layer 1010 and the wound contact layer 1030 can be similar to the top layers 810, 910 and the wound contact layers, 830, 930 described above in relation to Figs. 10A-12B.

The intermediate layer 1020 can be a masking or obscuring layer 1020 to help reduce the unsightly appearance of a drape 1000 during use due to the absorption of wound exudate. The obscuring layer 1020 may be a colored portion of the transmission layer 1040, or may be a separate layer that covers the transmission layer 1040. The obscuring layer 1020 may be one of a variety of colors such as blue, orange, yellow, green, or any color suitable for masking the presence of wound exudate in the drape 1000. For example, a blue obscuring layer 1020 may be a shade of blue similar to the shade of blue commonly used for the material of medical gowns, scrubs, and drapes. Some configurations of the obscuring layer 1020 may comprise polypropylene spunbond material. Further, some configurations of the obscuring layer 1020 may comprise a hydrophobic additive or coating. Some configurations may comprise a thin fibrous sheet of 60, 70, or 80 gsm.

The obscuring layer 1020 may comprise at least one viewing window configured to allow a visual determination of the saturation level of the transmission layer 1040. The at least one viewing window may comprise at least one aperture made through the obscuring layer 1020. The at least one viewing window may comprise at least one uncolored region of the obscuring layer 1020. Some configurations of the obscuring layer 1020 may comprise a plurality of viewing windows or an array of viewing windows.

The masking capabilities of the obscuring layer 1020 can be partial or less than 100% obscuring to thereby allow clinicians to access the information they require by observing the spread of exudate across the dressing surface. An obscuring layer 1020 may be partial due to material properties allowing wound exudate to slightly alter the appearance of the dressing or due to the presence of at least one viewing window in a completely obscuring material. The partial masking nature of the obscuring layer 1020 may enable a skilled clinician to perceive a different colour caused by exudate, blood, by-products etc. in the dressing allowing for a visual assessment and monitoring of the extent of spread across the dressing. However, since the change in colour of the dressing from its clean state to a state with exudate contained may only be a slight change, the patient is unlikely to notice any aesthetic difference. Reducing or eliminating a visual indicator of wound exudate from a patient is likely to have a positive effect on their health, reducing stress for example.

The transmission layer 1040 can be configured to act as a transmission layer similar to the intermediate layer 720 describe above in relation to Figs. 7-9. Additionally, the transmission layer 1040 can include a 3D fabric material or any of the materials described above for the intermediate layer 720. In some embodiments, the transmission layer may additionally or alternatively comprise an absorbent material, and in other embodiments, the transmission layer may not have any absorbent material. If absorbent material is utilized, the absorbent material may be a foam or non-woven natural or synthetic material and which may optionally include or be super-absorbent material forms a reservoir for fluid, particularly liquid, removed from the wound site. The material of the transmission layer 1040 also prevents liquid collected in the drape 1000 from flowing in a sloshing manner. The transmission layer 1040 can help distribute fluid throughout the layer 1040 via a wicking action so that fluid is drawn from the wound site and stored throughout the transmission layer 1040. This can help prevent agglomeration in areas of the transmission layer 1040. The capacity of the absorbent material must be sufficient to manage the exudates flow rate of a wound when negative pressure is applied. Since in use the transmission layer 1040 experiences negative pressures the material of the transmission layer 1040 is chosen to absorb liquid under such circumstances. A number of materials exist that are able to absorb liquid when under negative pressure, for example superabsorber material. In addition to materials described above, the transmission layer 1040 may be manufactured from or comprise ALLEVYN^{™} foam, Freudenberg 114-224-4 and/or Chem-Posite^{™} 11C-450.

In some configurations, the transmission layer 1040 can include non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers may lead to strong capillary action in the fibrous pad which helps distribute liquid. In this way, the super-absorbent material is efficiently supplied with liquid. Also, all regions of the transmission layer 1040 are provided with liquid. The wicking action also assists in bringing liquid into contact with the top layer 1010 to aid increase transpiration rates of the drape 1000. The wicking action may also assist in delivering liquid downwards towards the wound bed when exudation slows or halts. This delivery process can help maintain the transmission layer 1040 and lower wound bed region in a moist state which helps prevent crusting within the drape 1000, which could lead to blockages, and helps maintain an environment optimized for wound healing.

In some configurations, the transmission layer 1040 may include an air-laid material or any suitable material disclosed herein. Heat fusible fibers may optionally be used to assist in holding the structure of the drape 1000 together. It will be appreciated that rather than using super-absorbing particles or in addition to such use, super-absorbing fibers may be utilized according to certain configurations of the present disclosure. An example of a suitable material is the Product Chem-Posite^{™} 11 C available from Emerging Technologies Inc (ETi) in the USA.

Optionally, according to certain configurations of the present disclosure, the transmission layer 1040 may include synthetic stable fibers and/or bi-component stable fibers and/or natural stable fibers and/or super-absorbent fibers. Fibers in the transmission layer 1040 may be secured together by latex bonding or thermal bonding or hydrogen bonding or a combination of any bonding technique or other securing mechanism. In some configurations, the transmission layer 1040 may be formed by fibers which operate to lock super-absorbent particles within the transmission layer 1040. Such an arrangement can help ensure that super-absorbent particles do not move external to the transmission layer 1040 and towards an underlying wound bed. This may improve functionality because when negative pressure is applied there is a tendency for the transmission layer 1040 to collapse downwards and this action may push super-absorbent particle matter into a direction towards the wound bed if they were not locked away by the fibrous structure of the transmission layer 1040.

The transmission layer 1040 may comprise multiple fibers. The fibers can be strand-like and made from cellulose, polyester, viscose or the like. Dry absorbent particles can be distributed throughout the transmission layer 1040 ready for use. In some configurations, the transmission layer 1040 includes a pad of cellulose fibers and a plurality of super absorbent particles. In additional configurations, the transmission layer 1040 can be a non-woven layer of randomly orientated cellulose fibers.

The transmission layer 1040 may include a length L₁ and a width W₁. The length L₁ can be approximately 20 cm. The length L₁ can be between approximately 10 cm to approximately 100 cm, approximately 20 cm to approximately 90 cm, approximately 30 cm to approximately 80 cm, approximately 40 cm to approximately 70 cm, and approximately 50 cm to approximately 60 cm or any suitable length. The width W₁ can be approximately 20 cm. The width W₁ can be between approximately 10 cm to approximately 100 cm, approximately 20 cm to approximately 90 cm, approximately 30 cm to approximately 80 cm, approximately 40 cm to approximately 70 cm, and approximately 50 cm to approximately 60 cm or any suitable width.

As shown in Figs. 12A-13, the drape 1000 can include a drape tail 1000b. The drape tail 1000b can have a length and a width such that the width of the drape tail 1000b is smaller than the length. The width of the drape tail 1000b can be smaller than a width of the drape portion 1000a. The drape tail 1000b can include a proximal end 1002 and a distal end 1004. As shown in Fig. 12C, in some configurations, the top layer 1010 and the transmission layer 1040 of the drape portion 1000a can extend to form a top layer portion 1051 and a transmission layer portion 1053, respectively, of the drape tail 1000b. In some configurations, the top layer portion 1051 and the transmission layer portion 1053 of the drape tail 1000b can be continuous and integral with the top layer 1010 and the transmission layer 1040 of the drape portion 1000b. In some configurations, the top layer portion 1051 and the transmission layer portion 1053 of the drape tail 1000b can be continuous with the top layer 1010 and the transmission layer 1040 of the drape portion 1000a but comprise separate pieces of material from the top layer 1010 and the transmission layer 1040 of the drape portion 1000a.

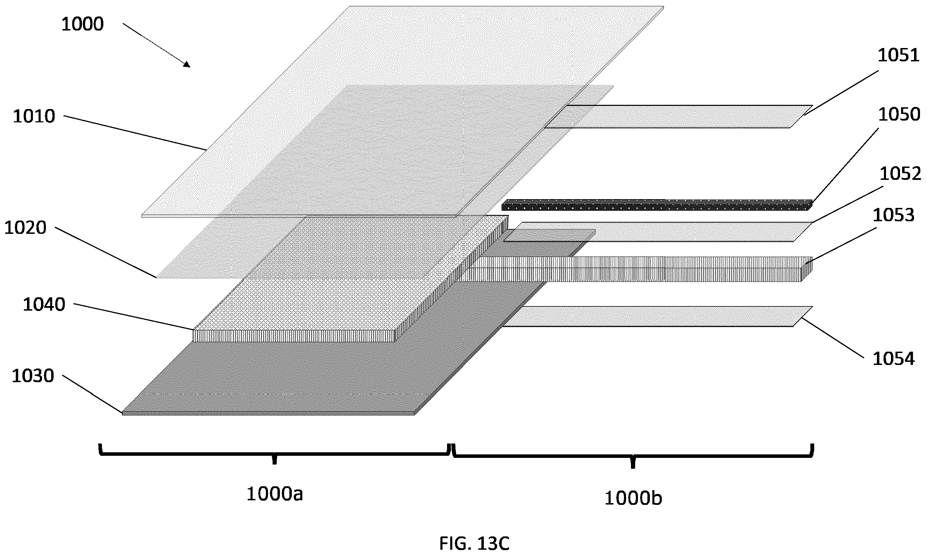

The top layer portion 1051 and the transmission layer portion 1053 of the drape tail 1000b can comprise elongate portions extending between the proximal and distal ends 1002, 1004 of the drape tail. The distal end 1004 of the drape tail 1000b can be positioned on a side (Figs. 12A-12C, for example centered on a side) or a corner (Fig. 13) of the drape portion 1000a. The proximal end 1002 of the drape tail 1000b can be configured to attach to a port that can be configured to apply negative pressure to the drape 1000, such as any port described herein this section or elsewhere in the specification, such as Figs. 4A-6. When negative pressure is applied to the drape 1000, the exudate can flow along a flow path 1060 defined by the transmission layer of the drape tail 1000b.

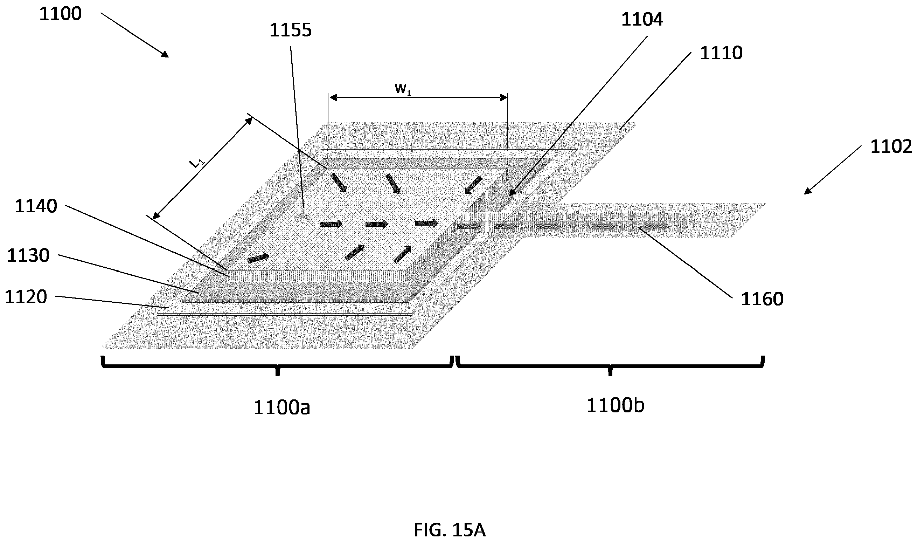

As illustrated in Figs. 12A-12C, in one embodiment a layer of porous material, such as a black foam layer, may be provided as part of the drape to provide a controlled leak path into the drape portion. For example, a black foam layer 1050 can be incorporated into the drape tail 1000b beneath the top layer portion 1051 to create an air leak 1055, for example at the proximal end 1002 thereof. As illustrated, the foam layer is elongated and may be extend over the transmission layer of the drape tail, and may extend over a portion of the transmission layer of the drape portion. In some configurations, the transmission layer 1040 of the drape tail 1000b can be sandwiched between a bottom layer 1054 and an intermediate layer 1052 while the black foam layer 1050 can be positioned between the intermediate layer 1052 and the top layer 1051 of the drape tail 1000b. The intermediate layer 1052 and the bottom layer 1054 can have elongate portions extending between the proximal and distal ends 1002, 1004 of the drape tail 1000b. The intermediate and bottom layers 1052, 1054 can comprise a material or materials that are fluid-impermeable, for example polymers such as polyurethane. It will of course be appreciated that the intermediate and bottom layers 1052, 1054 may each be constructed from different materials, including semi-impermeable materials. The bottom layer 1054 may optionally include an adhesive material on the skin-contacting surface such that the bottom layer 1054 can adhere to the user in use. Further details regarding the layers and materials that may be utilized in the drape tail 1000b are described above with respect to suction adapter 5500 and in U.S. Patent No. 8,801,685, including but not limited to Figs. 15A-15D and 55A-56C and the associated description, and U.S. Patent No. 9,907,703, including but not limited to Figs. 5A-5C and the associated description.

In some configurations, the air leak 1055 may comprise an opening or channel extending through top layer 1051, such that the air leak 1055 is in fluidic communication with the black foam layer 1050. Upon the application of suction to the drape tail 1000b, air will enter through the air leak 1055 and move from the proximal end 1002 to the distal end 1004 along the black foam layer 1050. The air will then be suctioned into the transmission layer 1040 by passing through the apertures throughout the transmission layer 1040.

In some configurations, the drape 1000 can maintain a constant leak rate through the air leak 1055 while negative pressure is applied through a source of negative pressure. Some configurations may support an air leak 1055 of about 1, 2, 3, 4, 5, 6, 7, 8, 9 mL/min or more (+/- .5 mL/min or another suitable deviation). Some configurations may support an air leak of 10, 20, 30, 40, 50, 60, 70, 80, 90 mL/min, or more (+/- a few mL/min or another suitable deviation). Some configurations may support an air leak of about 0.1, 0.2, 0.3, 0.4, 0.5, 0.6, 0.7, 0.8, 0.9 L/min, or more (+/- a few centiliters/min or another suitable deviation). In some instances, the leak rate can be discussed in terms of controlled leak pathways (CLPs), where CLP is a suitable constant. For instance, an air leak may have a leak rate of about 0.25, 0.5, 1, 2, 3, 4, 5, 6, 7, 8, 9, 10, or more. For example, assuming that a leak rate of 0.1 L/min, 1 CLP may correspond to 0.1 L/min, 5 CLPs may correspond to 0.5 L/min, and so on. The negative pressure source may be required to work harder in the presence of a higher intensity air leak, which can drain the power source faster. Thus, in some configurations, a relatively low leak rate can be chosen.

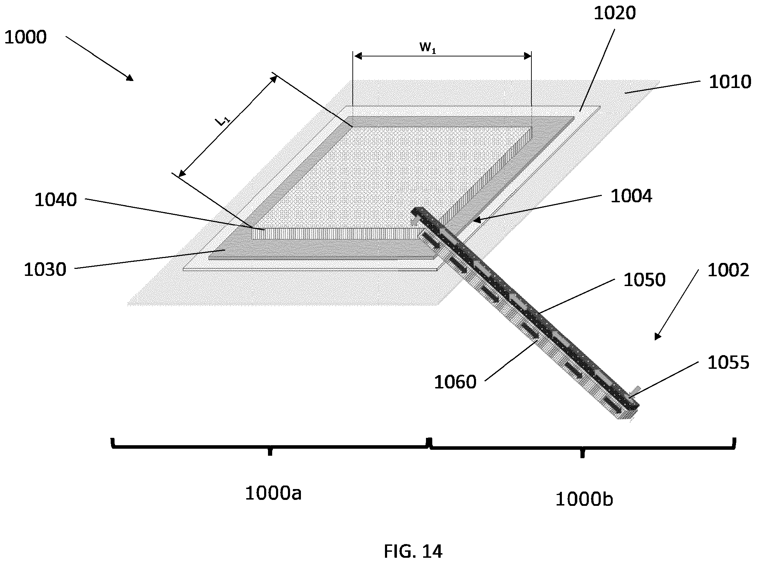

With reference to Figs. 14A-14C, another configuration of a drape 1100 is shown. The drape 1100 resembles or is identical to the drapes 700, 1000 discussed above in many respects. Any component or step disclosed in any configuration in this specification can be used in any other configuration.

Figs. 14A-14C shows a configuration of a drape 1100 that can be similar to the drape 1000 described above in relation to Figs. 12A-13. For example, the drape 1100 can include a drape portion 1100a with four layers and a drape tail 1100b. The drape portion 1100a may include a top layer 1110, an intermediate layer 1120, a wound contact layer 1130, and a transmission layer 1140. As shown in Figs. 14B-14C, the top layer 1110 can be positioned over the intermediate layer 1120 with the transmission layer 1140 being positioned between the intermediate layer 1120 and the wound contact layer 1130. Although Figs. 14A-14C show the drape 1100 with four layers and a certain positioning of the layers, the drape 1100 can include less than or more than four layers and the layers can be arranged in a different order.

The drape tail 1100b shown in Figs. 14A-14C can be similar to the drape tail 1000b described above in relation to Figs. 12A-13. In this embodiment, no foam layer is provided, but in other embodiments, a foam layer may be utilized. For example, the drape tail 1100b can include a proximal end 1102 and a distal end 1104 with the top layer 1151, the transmission layer 1153, and a bottom layer 1154 having elongate portions extending between the ends 1102, 1104. As shown in Fig. 14C, in some configurations, the transmission layer 1153 of the drape tail 1100b can be sandwiched between the bottom layer 1154 and the top layer 1151. The drape 1100 can include an air leak1155 positioned on the transmission layer 1140 opposite the drape tail 1100b. In some configurations, the air leak 1155 may comprise an opening or channel extending through top layer 1110, such that the air leak 1155 is in fluidic communication with the transmission layer 1140. Upon the application of suction to the drape tail 1100b, air will enter through the air leak 1155 and be suctioned into the transmission layer 1140 through the proximal end 1102 of the drape tail 1100b.

With reference to Figs. 15A-15C, another configuration of a drape 1200 is shown. The drape 1200 resembles or is identical to the drapes 700, 1000, 1100 discussed above in many respects. Any component or step disclosed in any configuration in this specification can be used in any other configuration.

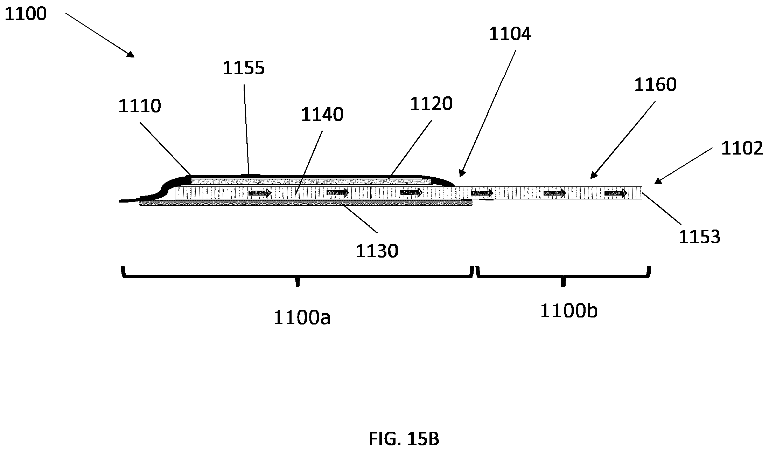

Figs. 15A-15C show a configuration of a drape 1200 that can be similar to the drapes 1000, 1100 described above in relation to Figs. 12A-14C. For example, the drape 1200 can include a drape portion 1200a with four layers and a drape tail 1200b. The drape portion 1200a may include a top layer 1210, an intermediate layer 1220, a wound contact layer 1230, and a transmission layer 1240. As shown in Figs. 15B-15C, the top layer 1210 can be positioned over the intermediate layer 1220 with the transmission layer 1240 being positioned between the intermediate layer 1220 and the wound contact layer 1230. The drape 1200 can include a plurality air leaks or CLPs 1255 positioned at various positions on the transmission layer 1240. As illustrated, air leaks or CLPS 1255 may be provided at one, more or all corners of the drape portion 1200a, and may also be provided along a side wall of the transmission layer 1240 opposite the drape tail 1200b. Although Figs. 15A-15C show the drape 1200 with four layers and a certain positioning of the layers, the drape 1200 can include less than or more than four layers and the layers can be arranged in a different order.

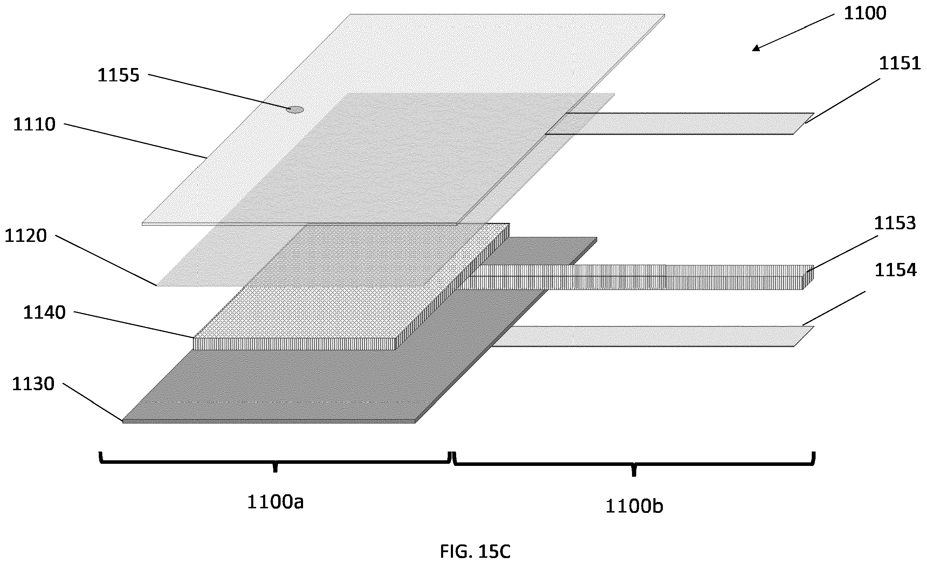

The drape tail 1200b shown in Figs. 15A-15C can be similar to the drape tails 1000b, 1100b described above in relation to Figs. 12A-14C. For example, the drape tail 1200b can include a proximal end 1202 and a distal end 1204 with the top layer 1251, the transmission layer 1253, and a bottom layer 1254 having elongate portions extending between the ends 1202, 1204. As shown in Fig. 15C, in some configurations, the transmission layer 1240 of the drape tail 1200b can be sandwiched between the bottom layer 1254 and the top layer 1210.

### Method of Treating a Wound with a Multilayer Drape Optionally Configured with an Integrated Port

Some configurations described herein provide a method of treating a wound using wound dressings and drapes as described herein, such as the wound dressings or drapes 1000, 1100, 1200 described above in relation to Figs. 12A-15C. In some configurations, the method of treating the wound may comprise applying a black foam material to the transmission layer 1040 of the drape tail 1000b. The drape portion 1000a, 1100a, 1200a of the drape 1000, 1100, 1200 can be positioned on the wound and the periwound area such that the top layer 1010, 1110, 1210 adheres to the periwound area and the wound contact layer 1030, 1130, 1230 adheres to the wound site. The drape tail 1000b, 1100b, 1200b can also be positioned on and/or adhered to the wound and the periwound area. The drape tail 1000b, 1100b, 1200b can be attached to a negative pressure applicator. Negative pressure can be applied to the wound and periwound area through the drape 1000, 1100, 1200 such that wound exudate can be removed from the wound and periowound area.

In some alternative configurations, the method of treating a wound may comprise using the drape 1000, 1100, 1200 under ambient conditions not in connection with a negative pressure wound therapy system as described above, or described elsewhere herein.

### Terminology

Aspects of the disclosure can be modified, if necessary, to employ the systems, functions, and concepts of the various references described herein to provide yet further implementations.

Features, materials, characteristics, or groups described in conjunction with a particular aspect, embodiment, or example are to be understood to be applicable to any other aspect, embodiment or example described herein unless incompatible therewith. All of the features disclosed in this specification (including any accompanying claims, abstract and drawings), or all of the steps of any method or process so disclosed, may be combined in any combination, except combinations where at least some of such features or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

While certain embodiments have been described, these embodiments have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some embodiments, the actual steps taken in the processes illustrated or disclosed may differ from those shown in the figures. Depending on the embodiment, certain of the steps described above may be removed, others may be added. For example, the actual steps or order of steps taken in the disclosed processes may differ from those shown in the figure. Depending on the embodiment, certain of the steps described above may be removed, others may be added. Furthermore, the features and attributes of the specific embodiments disclosed above may be combined in different ways to form additional embodiments, all of which fall within the scope of the present disclosure.

Although the present disclosure includes certain embodiments, examples and applications, it will be understood by those skilled in the art that the present disclosure extends beyond the specifically disclosed embodiments to other alternative embodiments or uses and obvious modifications and equivalents thereof, including embodiments which do not provide all of the features and advantages set forth herein. Accordingly, the scope of the present disclosure is not intended to be limited by the described embodiments but by the claims.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Likewise the term "and/or" in reference to a list of two or more items, covers all of the following interpretations of the word: any one of the items in the list, all of the items in the list, and any combination of the items in the list. Further, the term "each," as used herein, in addition to having its ordinary meaning, can mean any subset of a set of elements to which the term "each" is applied. Additionally, the words "herein," "above," "below," and words of similar import, when used in this application, refer to this application as a whole and not to any particular portions of this application.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

## Claims

1. A system (5501) to provide negative pressure to a wound site and a periwound area surrounding the wound site comprising:
a wound dressing (700) comprising a top layer (710), an intermediate layer (720) and a wound contact layer (730), wherein each layer is constructed from a flexible, liquid impermeable material, and wherein each of the layers comprises an upper surface and a lower surface; wherein
the top layer defines a perimeter configured to be positioned over the wound site and the periwound area;
the intermediate layer is positioned between the top layer and the wound contact layer, wherein the intermediate layer has a smaller footprint than the top layer and the wound contact layer; and
the wound contact layer is positioned below the intermediate layer and is configured to contact the wound site and the periwound area, wherein the wound contact layer comprises a plurality of apertures; and
a port (740) configured to be secured to the upper surface of the top layer and to apply negative pressure through the top layer for the application of topical negative pressure at the wound site and the periwound area, wherein the top layer includes a hole (716) configured to receive the port, wherein the upper surface of the top layer comprises one or more placement guides (715) configured to aid a user in aligning the port to the hole.

2. The system of Claim 1, wherein the one or more placement guides are printed directly on the upper surface of the top layer.

3. The system of Claim 1, wherein the one or more placement guides are provided on a sticker that is configured to be attached to the upper surface of the top layer.

4. The system of any one of Claims 1-3, wherein the one or more placement guides comprises two substantially perpendicular lines that intersect at the opening.

5. The system of any one of Claims 1-4, wherein the one or more placement guides comprises a circle with the opening at a center of the circle.

6. The system of any one of Claims 1-5, wherein the port comprises one or more alignment features that are configured to interact with the one or more placement guides on the upper surface of the top layer.

7. The system of any one of Claims 1-6, wherein the intermediate layer comprises an acquisition distribution layer configured to communicate fluid through the wound dressing.

## Patentansprüche

1. Ein System (5501) zur Bereitstellung von Unterdruck an einer Wundstelle und einem die Wundstelle umgebenden Wundumgebungsbereich, das Folgendes beinhaltet:
einen Wundverband (700), der eine oberste Schicht (710), eine Zwischenschicht (720) und eine Wundkontaktschicht (730) beinhaltet, wobei jede Schicht aus einem flexiblen,
flüssigkeitsundurchlässigen Material aufgebaut ist und wobei jede der Schichten eine obere Fläche und eine untere Fläche beinhaltet; wobei
die oberste Schicht einen Umfang definiert, der konfiguriert ist, um über die Wundstelle und den Wundumgebungsbereich positioniert zu sein;
die Zwischenschicht zwischen der obersten Schicht und der Wundkontaktschicht positioniert ist, wobei die Zwischenschicht eine kleinere Stellfläche als die oberste Schicht und die Wundkontaktschicht aufweist; und
die Wundkontaktschicht unter der Zwischenschicht positioniert ist und konfiguriert ist, um die Wundstelle und den Wundumgebungsbereich zu kontaktieren, wobei die Wundkontaktschicht eine Vielzahl von Öffnungen beinhaltet; und
einen Anschluss (740), der konfiguriert ist, um an einer oberen Fläche der obersten Schicht gesichert zu werden und Unterdruck durch die oberste Schicht für die Anwendung von topischem Unterdruck an der Wundstelle und dem Wundumgebungsbereich anzuwenden, wobei die oberste Schicht ein Loch (716) umfasst, das konfiguriert ist, um den Anschluss aufzunehmen, wobei die obere Fläche der obersten Schicht eine oder mehrere Platzierungsführungen (715) beinhaltet, die konfiguriert sind, um einen Benutzer bei der Ausrichtung des Anschlusses auf das Loch zu unterstützen.

2. System gemäß Anspruch 1, wobei die eine oder mehrere Platzierungsführungen auf die obere Fläche der obersten Schicht gedruckt sind.

3. System gemäß Anspruch 1, wobei die eine oder mehrere Platzierungsführungen auf einem Aufkleber bereitgestellt sind, der konfiguriert ist, um auf der oberen Fläche der obersten Schicht angebracht zu werden.

4. System gemäß einem der Ansprüche 1-3, wobei die eine oder mehreren Platzierungsführungen zwei im Wesentlichen senkrechte Linien, die sich an der Öffnung kreuzen, beinhaltet.

5. System gemäß einem der Ansprüche 1-4, wobei die eine oder mehreren Platzierungsführungen einen Kreis beinhaltet, wobei die Öffnung in der Mitte des Kreises liegt.

6. System gemäß einem der Ansprüche 1-5, wobei der Anschluss eine oder mehrere Ausrichtungseinrichtungen beinhaltet, die konfiguriert sind, um mit der einen oder den mehreren Platzierungsführungen auf der oberen Fläche der obersten Schicht zusammenzuwirken.

7. Einrichtung gemäß einem der Ansprüche 1-6, wobei die Zwischenschicht eine Erfassungsverteilungsschicht beinhaltet, die konfiguriert ist, um Flüssigkeit durch den Wundverband zu kommunizieren.

## Revendications

1. Un système (5501) destiné à fournir une pression négative à un site de plaie et à une zone périplaie entourant le site de plaie comprenant :
un pansement pour plaie (700) comprenant une couche de dessus (710), une couche intermédiaire (720) et une couche en contact avec la plaie (730), où chaque couche est construite à partir d'un matériau souple imperméable aux liquides, et où chacune des couches comprend une surface supérieure et une surface inférieure ; où
la couche de dessus définit un périmètre configuré pour être positionné pardessus le site de plaie et la zone périplaie ;
la couche intermédiaire est positionnée entre la couche de dessus et la couche en contact avec la plaie, où la couche intermédiaire présente une plus petite zone de couverture que la couche de dessus et la couche en contact avec la plaie ; et
la couche en contact avec la plaie est positionnée en dessous de la couche intermédiaire et est configurée pour venir en contact avec le site de plaie et la zone périplaie, où la couche en contact avec la plaie comprend une pluralité de trous ; et
un port (740) configuré pour être assujetti à la surface supérieure de la couche de dessus et pour appliquer une pression négative à travers la couche de dessus pour l'application de pression négative topique au niveau du site de plaie et de la zone périplaie, où la couche de dessus inclut un orifice (716) configuré pour recevoir le port, où la surface supérieure de la couche de dessus comprend un ou plusieurs guides de placement (715) configurés pour aider un utilisateur à aligner le port sur l'orifice.

2. Le système de la revendication 1, où les un ou plusieurs guides de placement sont imprimés directement sur la surface supérieure de la couche de dessus.

3. Le système de la revendication 1, où les un ou plusieurs guides de placement sont prévus sur un autocollant qui est configuré pour être fixé sur la surface supérieure de la couche de dessus.

4. Le système de l'une quelconque des revendications 1 à 3, où les un ou plusieurs guides de placement comprennent deux lignes substantiellement perpendiculaires qui se coupent au niveau de l'ouverture.

5. Le système de l'une quelconque des revendications 1 à 4, où les un ou plusieurs guides de placement comprennent un cercle, l'ouverture étant au niveau d'un centre du cercle.

6. Le système de l'une quelconque des revendications 1 à 5, où le port comprend un ou plusieurs éléments d'alignement qui sont configurés pour interagir avec les un ou plusieurs guides de placement sur la surface supérieure de la couche de dessus.

7. Le système de l'une quelconque des revendications 1 à 6, où la couche intermédiaire comprend une couche d'acquisition-distribution configurée pour communiquer du fluide à travers le pansement pour plaie.
